# EUROPEAN PATENT APPLICATION

(11) **EP 4 397 677 A1**
(43) Date of publication of application: **10.07.2024**
(21) Application number: 22863257.6
(22) Date of filing: 24.08.2022
(51) Int. Cl.: C07K 14/55, A61K 38/20, A61K 47/60, A61P 35/00, A61P 35/02, A61P 37/04

(54) **HUMAN INTERLEUKIN 2-POLYETHYLENE GLYCOL CONJUGATE AND USE THEREOF**

(30) Priority: 01.09.2021 CN 202111019771
(71) Applicant: Novocodex Biopharmaceuticals Co., Ltd., Shaoxing, Zhejiang 312366 (CN)
(72) Inventor: LIANG, Xuejun, Shaoxing, Zhejiang 312366 (CN); YE, Chenghao, Shaoxing, Zhejiang 312366 (CN); XIA, Gang, Shaoxing, Zhejiang 312366 (CN); CHEN, Longfei, Shaoxing, Zhejiang 312366 (CN); HUO, Pengchao, Shaoxing, Zhejiang 312366 (CN); YANG, Jinwei, Shaoxing, Zhejiang 312366 (CN); YING, Yuebin, Shaoxing, Zhejiang 312366 (CN); GONG, Zunyang, Shaoxing, Zhejiang 312366 (CN); HENG, Xin, Shaoxing, Zhejiang 312366 (CN); ZHU, Xiaoyu, Shaoxing, Zhejiang 312366 (CN); ZHU, Lifei, Shaoxing, Zhejiang 312366 (CN); HUANG, Hao, Shaoxing, Zhejiang 312366 (CN); DING, Wen, Shaoxing, Zhejiang 312366 (CN); JIAO, Lin, Shaoxing, Zhejiang 312366 (CN); ZHU, Jingjing, Shaoxing, Zhejiang 312366 (CN)
(74) Representative: Aera A/S
(86) International application number: PCT/CN2022/114421
(87) International publication number: WO 2023/030118

(57) **Abstract**

A human interleukin 2-polyethylene glycol conjugate and use thereof. The human interleukin 2-polyethylene glycol conjugate comprises a recombinant human interleukin 2 containing at least one unnatural amino acid and PEG coupled to the at least one unnatural amino acid. The unnatural amino acid is a compound containing a carbonyl terminal group and having a structure as shown in formula (I) or an enantiomer thereof, and the PEG is coupled to the at least one unnatural amino acid by forming an oxime bond between the carbonyl terminal group and the PEG containing a hydroxylamine terminal group. The human interleukin 2-polyethylene glycol conjugate can be used individually or in combination with other anti-tumor drugs for the treatment of diseases such as solid tumors and hematologic tumors.

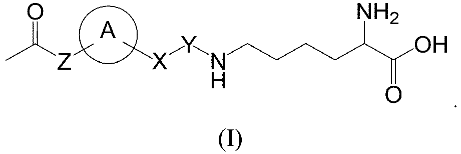

## Description

### TECHNICAL FIELD

The present invention relates to the field of biopharmaceuticals, and particularly relates to a human interleukin 2-polyethylene glycol conjugate and use thereof.

### BACKGROUND

Interleukin 2 (IL-2) is an important immunoregulatory factor produced from activated type-I helper T lymphocytes (Th1), which was once called a T cell growth factor, with a main biological function of promoting the growth, proliferation and differentiation of T cells (comprising CD4⁺ and CD8⁺ T cells) by dual ways of stimulation and anti-apoptosis, and promoting the further secretion of cytokines. In addition, interleukin 2 also stimulates the proliferation of NK cells, enhances the killing activity of NK and produces the cytokines, and induces the production of LAK cells; and promotes the proliferation of B cells and secretion of antibodies. Therefore, interleukin 2 plays an important role in immune response and antiviral infection (Gaffena S.L, Cytokine 28: 109e123, 2004).

IL-2 has been widely used in clinic since it was first discovered by Morgan et al. in 1976. In 1991, rhIL-2 (product name: Aldesleukin) produced by Cetus in the United States was approved by FDA, which was widely used in malignant tumors such as renal cell carcinoma, malignant melanoma and malignant lymphoma (the instruction of Proleukin), and also had potential effects in the adjuvant treatment of hepatitis B and hepatitis C infection (Tomova R. et al., Anticancer Research, 29:5241-5244, 2009). Up to now, more than 10 recombinant human interleukin 2 biological products have been put into production and listed in China, which are widely used in the treatment of malignant tumors such as renal cell carcinoma, melanoma, breast cancer, bladder cancer, liver cancer, rectal cancer, lymphoma and lung cancer, used for the control of cancerous hydrothorax and ascites, used for the enhancement of immune function of tumor patients after surgery, radiotherapy and chemotherapy, used for the improvement of cellular immune function and anti-infection ability of patients with congenital or acquired immunodeficiency, and used for the treatment of various autoimmune diseases such as rheumatoid arthritis, systemic lupus erythematosus, and Sjogren syndrome, and have a certain therapeutic effect on some viral, bacillary and intracellular bacterial parasitic infectious diseases, such as hepatitis B, leprosy, tuberculosis and Candida albicans infection.

The precursor of human IL-2 is composed of 153 amino acid residues, and when the precursor is secreted out of cells, the signal peptide (comprising 20 amino acid residues) of the precursor is excised, and a mature IL-2 with 133 amino acids is produced, with a molecular weight of 15.4 kD. The activating effect of IL-2 on effector cells is realized by binding with an IL-2 receptor (IL-2R) on a cell surface. It has been found that the IL-2 receptor comprises IL-2Rα, IL-2Rβ and IL-2Rγ, which may form a heteromultimeric glycoprotein functional complex IL-2Rαβγ with high-affinity (Kd=10⁻¹¹ mol/L). IL-2Rβ and IL-2Rγ may form a receptor complex IL-2Rβγ with moderate-affinity (Kd=10⁻⁹ mol/L), which has biological activity after being activated by IL-2. IL-2Rα subunit is in a form of low-affinity receptor (Kd=10⁻⁸ mol/L), which cannot transmit an intracellular proliferation signal when binding with IL-2. Although IL-2Rα and IL-2Rβ may also form a receptor complex with high-affinity, the receptor complex have no biological function and cannot be activated by IL-2. In different cells, different developmental stages of the same type of cells and different states of diseases, types of the IL-2 receptor have different expression degrees, thereby forming different receptor complexes. For example, LAK cell precursors express a high-level IL-2Rβγ complex, and may attack and lyse cancer cells after being activated by IL-2. Macrophages also express the IL-2Rβγ complex, and may be activated by IL-2. Monocytes express a large amount of IL-2Rγ and a small amount of IL-2Rβ, while NK cells express a large amount of IL-2Rβ and a small amount of IL-2Rγ, which form IL-2Rβγ receptors with moderate-affinity respectively to bind with the high-concentration IL-2 to form trimers, and then the monocytes or the NK cells are activated. Activated T cells express IL-2Rα, IL-2Rβ and IL-2Rγ on their surfaces, excessive IL-2Rα is beneficial for polymerizing with IL-2Rβ first, and IL-2Rαβ binds with IL-2 and then binds with IL-2Rγ to form the high-affinity receptor IL-2 complex, thus transmitting a signal, and causing a cell proliferation reaction. After the cell reaction, IL-2Rα, IL-2Rβ and IL-2Rγ are dissociated, and cells are no longer sensitive to IL-2. Human tumor cells also express the IL-2 receptor, and IL-2 may inhibit the proliferation of tumor cells after binding with receptor complexes on the tumor cells. Because different cancer cells express respective special IL-2 receptor complexes, the structure of IL-2 is improved to act on the corresponding receptors on surfaces of specific tumor surfaces only, so that only cancer cells are attacked, and the damage to normal cells is reduced.

Based on this research theory, many researchers have modified IL-2 in different directions to enhance the binding with specific receptor complexes (such as the IL-2Rβγ complex) on surfaces of tumor-related effector cells, activate cell types related to tumor killing, and minimize the binding with the high-expression IL-2Rαβγ complexes on surfaces of negative immunoregulatory T cells (such as Treg cells) at the same time, which can not only enhance the efficacy of drugs, but also reduce the side effects of drugs. Existing modifications of IL-2 comprise: designing specific IL-2 mutant proteins (for example, Aron M.L. et al., Nature, 484(7395):529-33, 2012), and changing the amino acid sequence of the binding site with IL-2α, IL-2β or IL-2γ to make the spatial structure unfavorable to the interaction with IL-2α, or enhancing the interaction with IL-2Rβ or IL-2Rγ; designing an IL-2/anti-IL-2 antibody (or IL-2 receptor) complex (for example, Jared E.L. et al., Jimunother Cancer, 8(1): e000673, 2020), and using anti-IL-2 antibody to cover the binding site with IL-2R specifically, so as to realize the change of IL-2 function and the prolongation of in-vivo half-life period; carrying out fusion expression of IL-2 with Fc or human serum albumin (HSA) to prolong the in-vivo half-life period of IL-2 (for example, JianyongLei et al., Protein Expression and Purification, 84(1): 154-160, 2012); combining site-directed mutation with HSA/Fc fusion to simultaneously realize the change of function and the prolongation of half-life period (for example, CN112724259A); and carrying out PEGylation of IL-2 by non-site-directed coupling to prolong the half-life period of IL-2 (for example, Deborah H.C et al., Clin Cancer Res, 22(3):680-90, 2016).

The above researches on the modification of IL-2 have the following defects.
1. Simple site-directed mutation of amino acid may weaken the binding ability with IL-2Rα, or enhance the binding ability with IL-2Rβ or IL-2Rγ, but cannot effectively prolong the half-life period of molecules. Moreover, mutant products are easy to produce an immunogenic reaction in vivo, which easily reduces the biological activity of the products, and generates greater toxicity risks.
2. Simple fusion expression (such as fusion with Fc or HSA) or IL-2 modification can prolong the half-life period of molecules, but has no obvious advantages compared with unmodified IL-2 in practical use. Fusion expression can only be realized by modification of a fusion molecule at the N-terminal or the C-terminal of the target protein, and cannot realize the optimization of the modification site.
3. Some of IL-2 subjected to site-directed mutation combined with fusion expression does not show special advantages in practical application. (For example, Rodrigo Vazquez-Lombardi et al., Nat Commun, 8:15373, 2017).
4. Conventional PEGylated IL-2 by non-site-directed coupling does not show special advantages in practical application, and PEGylated IL-2 comprehensively weakening the binding ability with IL-2Rα achieves staged success, but the characteristics of non-site-directed coupling technology make the technology have the defects of difficult control of production process and quality, complicated molecular structure and complicated mechanism of action.

In view of the limitations of the IL-2 modifications above, some researchers have developed PEG site-directed coupled IL-2 (for example, WO 2019028419A1) by a codon expansion technology with an unnatural amino acid of Lys-azido, and the structural formula is as follows:

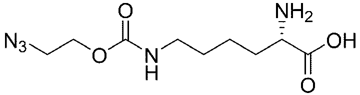

An azide structure (-N₃) at the end of the Lys-azido can be chemically linked with a carrier drug (such as PEG) modified with an alkyne-containing structure (such as BCN, i.e. 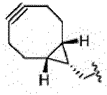 ) to obtain a conjugate (for example, Chinese patent CN 103153927B), which has very high specific selectivity. However, alkyne structures with high costs need to be introduced in this coupling method and chemical modification method, and an acceptable drug-antibody coupling ratio may be obtained only when an equivalent weight of use is large, which increases corresponding production costs, and has a complicated technological process and harsh technological conditions.

To sum up, there are still many shortcomings in current IL-2 modifications, and further researches are needed.

### SUMMARY

Aiming at the defects in the existing modifications of IL-2, one object of the present invention is to provide a human interleukin 2-polyethylene glycol conjugate, which uses a series of brand-new unnatural amino acids to mutate one or more natural amino acids in the amino acid sequence of the recombinant human interleukin-2, and then couples polyethylene glycol (PEG) to the unnatural amino acids in a site-directed manner through an oximation reaction, thereby forming the conjugate of the present invention.

Another object of the present invention is to provide use of the human interleukin 2-polyethylene glycol conjugate. The human interleukin 2-polyethylene glycol conjugate provided by the present invention may be used for treating diseases such as malignant solid tumors and hematologic tumors.

In a first aspect, the present invention provides a human interleukin 2-polyethylene glycol conjugate, comprising a recombinant human interleukin 2 containing at least one unnatural amino acid and PEG coupled to the at least one unnatural amino acid;
wherein, the unnatural amino acid is a compound containing a carbonyl terminal group and having a structure as shown in formula (I) or an enantiomer thereof, and the PEG is coupled to the at least one unnatural amino acid by forming an oxime bond between the carbonyl terminal group and the PEG containing a hydroxylamine terminal group (i.e., aminoxy), 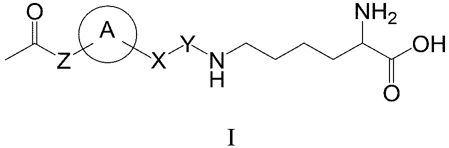
wherein X and Z each independently represent substituted or unsubstituted C0-C20 linear or branched alkylene, one or more -CH₂- are optionally substituted by one or more of -O-, -S-, -NH-, -C(O)- and -S(O)-, Y represents -C(O)-, -S(O)- or -CH₂-, and A represents substituted or unsubstituted C6-C20 aryl; and
when X, Z and A each independently represent a substituent, the substituent is selected from one or more of hydroxyl, sulfhydryl, halogen, nitro, cyano, alkyl, alkenyl, alkynyl, alkoxy, acyl, acylamino, carboxyl, ester, amino, sulfonyl, sulfinyl, cycloalkyl, heterocyclyl, aryl and heteroaryl.

As shown in Example 9, the inventor of the present invention found that, besides the high cost and the complicated process, the azide structure (-N₃) at the end of Lys-azido could be easily reduced into an amino structure (-NH₂) (as shown in formula 1) when the azide structure was inserted into a recombinant human interleukin 2, thus losing coupling activity, so that the reduction reaction reduces yield in a conjugate preparation process.

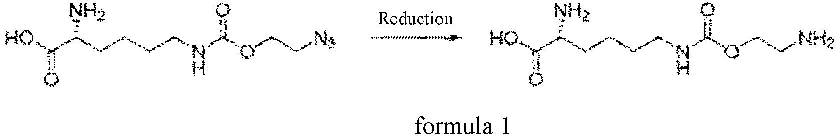

Carbonyl is introduced at the end of the unnatural amino acid according to the present invention as an active reaction group, so that the unnatural amino acid is not only novel in structure, simple and convenient to prepare, but also mild in coupling conditions and low in production cost, and is not easy to lose reaction activity due to a structural change when being inserted into a protein; and the unnatural amino acid according to the present invention further contains aryl connected with the carbonyl, the introduction of the aryl can further improve a stability of the obtained conjugate, and the conjugate is not easy to be decomposed even under a lower pH condition. In addition, the unnatural amino acid according to the present invention further contains alkylene with a certain chain length, so that the compound has better flexibility and is easier to form conjugates.

In the conjugate provided by the present invention, the unnatural amino acid contained in the recombinant human interleukin 2 contains a carbonyl terminal group, while the used PEG contains a hydroxylamine terminal group, having a structure as shown in formula (II), 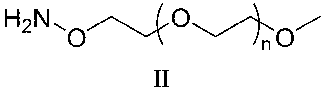 the carbonyl in the unnatural amino acid can be oximated with the hydroxylamine group in the PEG to form an oxime bond, with a structure as shown in formula (III), thus coupling the PEG to the unnatural amino acid. 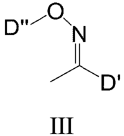 in formula (III), D' represents a residue of the recombinant human interleukin-2 of the present invention from which the carbonyl part of the unnatural amino acid is removed, and D" represents the PEG from which the "NH₂-O-" terminal group is removed.

Compared with the wild-type IL-2 or the commercially available recombinant human IL-2, the conjugate provided by the present invention has a reduced binding force with IL-2Rα, retains a binding activity with IL-2Rβγ, retains an ability to activate and expand CD8⁺ T cells through the activation of the CD8⁺ T cells by an IL-2Rβγ complex, inhibits the expansion of Treg cells at the same time, has a significantly prolonged half-life in vivo, and can effectively promote immunity and inhibit tumors. Moreover, the conjugate provided by the present invention has higher coupling rate and better stability.

In some preferred embodiments of the present invention, the recombinant human interleukin 2 is a protein shown in SEQ ID NO: 3 or a functional active fragment thereof.

In some preferred embodiments of the present invention, in the recombinant human interleukin 2 containing at least one unnatural amino acid, a position of the at least one unnatural amino acid is selected from one or more sites corresponding to sites P34, K35, T37, R38, L40, T41, F42, K43, F44, Y45, E61, E62, K64, P65, E67, E68, N71, L72 and Y107 of SEQ ID NO: 2. In some more preferred embodiments of the present invention, in the recombinant human interleukin 2 containing at least one unnatural amino acid, the position of the at least one unnatural amino acid is selected from one or more sites corresponding to sites K35, T41, K43, Y45, E61, K64 and P65 of SEQ ID NO: 2.

In the unnatural amino acid according to the present invention, "C0-Cn" includes C0-C1, C0-C2, ..., C0-Cn, and C0 indicates that the group does not exist, and C atoms at two ends of the group are directly connected to form a bond. For example, the "C0-C6" group refers to that there are 0 to 6 carbon atoms in this part, which means that the group does not exist, the group contains 1 carbon atom, 2 carbon atoms, 3 carbon atoms, 4 carbon atoms, 5 carbon atoms or 6 carbon atoms. The "C6-C10" group refers to that there are 6 to 10 carbon atoms in this part, which means that the group contains 6 carbon atoms, 7 carbon atoms, 8 carbon atoms, 9 carbon atoms or 10 carbon atoms.

In the unnatural amino acid according to the present invention, the "aryl" refers to a carbocyclic aromatic system containing one or two rings, wherein the rings may be connected together in a fused manner. The "aryl" includes monocyclic or bicyclic aryl, such as aromatic groups of phenyl, naphthyl and tetrahydronaphthyl. Preferably, the aryl is C6-C10 aryl, more preferably, the aryl is phenyl and naphthyl, and most preferably, the aryl is phenyl.

In some preferred embodiments of the present invention, the substituent may be selected from one or more of hydroxyl, sulfhydryl, halogen, nitro, cyano, C1-C6 alkyl, C1-C6 alkoxy, acyl, acylamino, carboxyl, ester, amino, sulfonyl, sulfinyl, C3-C8 cycloalkyl, C3-C8 heterocyclyl, C6-C20 aryl and C4-C10 heteroaryl.

In some preferred embodiments of the present invention, X and Z may each independently represent C0-C10 linear or branched alkylene, wherein one or more -CH₂- may be optionally substituted by one or more of -O-, -S- and -NH-. In some more preferred embodiments of the present invention, X and Z may each independently represent C0-C6 linear alkylene, wherein one or more -CH₂- may be optionally substituted by one or more of -O-, -S- and -NH-. In some more preferred embodiments of the present invention, X and Z are not C0 alkylene simultaneously, which means that the X and Z groups cannot exist simultaneously.

In some preferred embodiments of the present invention, A may represent substituted or unsubstituted C6-C10 aryl, and more preferably, A may represent substituted or unsubstituted phenyl or naphthyl.

In some preferred embodiments of the present invention, the unnatural amino acid may be a compound with a structure as shown in formula (I-1), 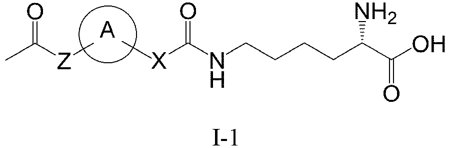 wherein X, Y and A are each independently as defined in any one of the above technical solutions.

In some preferred embodiments of the present invention, the unnatural amino acid may be a compound with a structure as shown in formula (I-2), 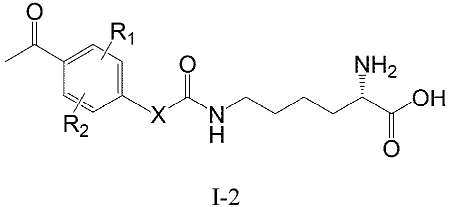
wherein X is as defined in any one of the above technical solutions; and
R₁ and R₂ each independently represent hydrogen, hydroxyl, sulfhydryl, halogen, nitro, cyano, C1-C6 alkyl, C1-C6 alkoxy, acyl, acylamino, carboxyl, ester, amino, sulfonyl, sulfinyl, C3-C8 cycloalkyl, C3-C8 heterocyclyl, C6-C20 aryl or C4-C10 heteroaryl.

In some preferred embodiments of the present invention, the unnatural amino acid is a compound with a structure as shown in formula (I-3), formula (I-4), formula (I-5) or formula (I-6), 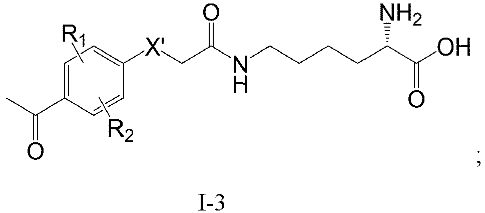 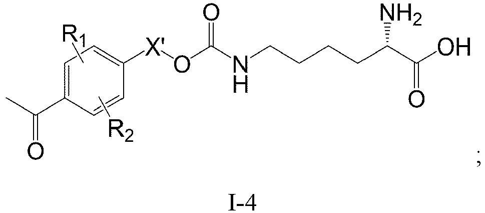 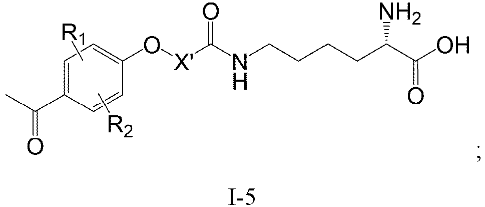 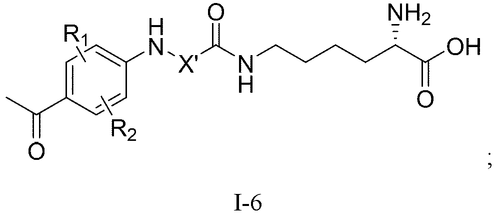
wherein X' represents C0-C6 linear alkylene, and more preferably represents C0-C4 linear alkylene, and one or more -CH₂- are optionally substituted by -O- and/or -NH-; and
R₁ and R₂ are each independently as defined in any one of the above technical solutions.

The unnatural amino acid according to the present invention includes optically pure enantiomer and racemate.

In some more preferred embodiments of the present invention, the unnatural amino acid according to the present invention is a compound with one of the structures as follows:

| | |
|---|---|
| (1) NBOK | 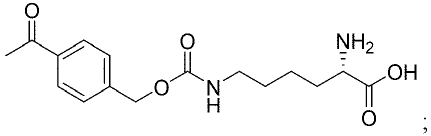 |
| (2) NPAK | 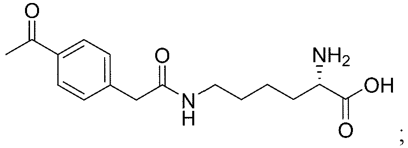 |
| (3) NBPK | 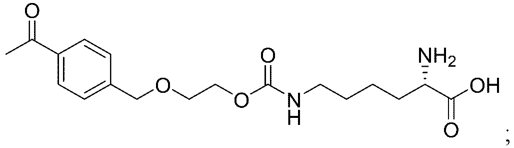 |
| (4) NPOK | 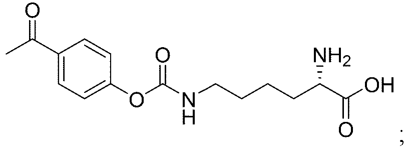 |
| (5) NBGK | 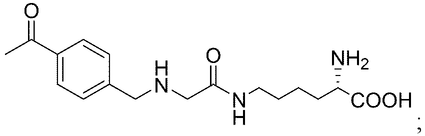 |
| (6) NPOK-2 | 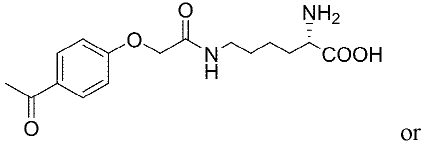 |
| (7) NBGK-2 | 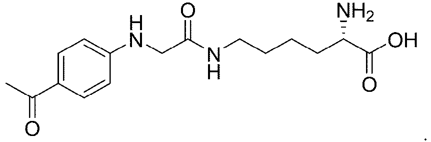 |

In some preferred embodiments of the present invention, the PEG containing the hydroxylamine ("NH₂-O-") terminal group may have a molecular weight of 10 KD to 100 KD, including, but being not limited to molecular weight values of about 10 KD, 20 KD, 30 KD, 40 KD, 50 KD, 60 KD, 70 KD, 80 KD, 90 KD, 100 KD or a molecular weight interval of any combination. In some more preferred embodiments of the present invention, the molecular weight of the PEG containing the "NH₂-O-" terminal group may be 20 KD to 50 KD.

In some preferred embodiments of the present invention, the recombinant human interleukin 2 containing at least one unnatural amino acid according to the present invention may be prepared by a codon expansion technology or by means of chemical synthesis. In some more preferred embodiments of the present invention, the recombinant human interleukin 2 containing at least one unnatural amino acid according to the present invention is prepared by the codon expansion technology, wherein the codon expansion technology is implemented in *Escherichia coli.*

The codon expansion technology according to the present invention may specifically include the following steps: comparing the mutated nucleic acid molecule of the recombinant human interleukin 2 with the nucleic acid molecule encoding the recombinant human interleukin 2, the mutated nucleic acid molecule is different in that: the codons of the amino acid corresponding to at least one sites of P34, K35, T37, R38, L40, T41, F42, K43, F44, Y45, E61, E62, K64, P65, E67, E68, N71, L72 and Y107 of SEQ ID NO: 2 are mutated into amber codons UAG; the mutated nucleic acid molecule is expressed in *Escherichia coli,* and meanwhile, a lysine analogue (such as NBOK) containing the carbonyl of the present invention is incorporated into the expressed recombinant human interleukin 2 through an orthogonal tRNA synthetase /tRNA pair. The working principle of the codon expansion system is as follows: tRNA^{Pyl} cannot use lysyl tRNA enzyme of host cells, but can only be acylated by tRNA^{Pyl} RS; tRNA^{Pyl} RS can only acylate tRNA^{Pyl}, but cannot acylate other tRNAs, that is, there is orthogonality between tRNA^{Pyl} and tRNA^{Pyl} RS, and only tRNA^{Pyl} RS can acylate the corresponding unnatural amino acids to this orthogonal tRNA, and can only acylate this tRNA, but cannot acylate other tRNAs. The codon expansion system can make the lysine analogues containing the carbonyl correspond to the amber codons UAG (that is, the codons corresponding to tRNA^{Pyl} are UAG), so that the lysine analogues containing the carbonyl can be introduced into IL-2 at a specific site.

In some preferred embodiments of the present invention, after expressing the mutated recombinant human interleukin 2 in *Escherichia coli,* steps of protein denaturation, renaturation and ultrafiltration are also included.

In some preferred embodiments of the present invention, the oximation reaction of the unnatural amino acid in the recombinant human interleukin 2 with the PEG containing the hydroxylamine may include the following process: before the oximation reaction, adjusting the pH of the mutated recombinant human interleukin 2 solution to about 3.5 to 4.5 (for example, the pH is adjusted to about 4.0 using 2M acetic acid solution), and adjusting the protein concentration to 0.5 mg/ml to 1.5 mg/ml (for example, using 20 mM sodium acetate buffer at pH 4.0), feeding PEG and protein according to a certain molar ratio (for example, protein: PEG = 1:15), fully dissolving, blocking, and shaking in a constant temperature shaker for reaction (for example, reaction for 30 hours to 60 hours). After the reaction, the coupling progress may be analyzed by a conventional detection method (for example, RP-HPLC).

In some preferred embodiments of the present invention, after the oximation reaction (i.e. the coupling reaction), the reaction solution contains some unreacted IL-2, impurity proteins and unreacted PEG, so the reaction solution may be further purified by cation exchange chromatography. For example, the following purification process may be adopted: chromatographic medium: Capto MMC; balance buffer: 20 mM sodium citrate buffer (pH = 3.0), elution buffer: 20 mM sodium citrate buffer -1 M NaCl (pH = 7.8), wherein the pH of the coupling reaction solution is adjusted to 3.0±0.2 with the balance buffer, and the conductivity is equal to or less than 5 ms/cm, then the reaction solution is loaded to Capto MMC to subject linear elution with the elution buffer (0% to 100% elution buffer, 20 CV) to collect target protein components. A target protein sample with a purity of about 95% can be obtained by this purification process.

In a second aspect, the present invention also provides use of the human interleukin 2-polyethylene glycol conjugate according to any one of the above technical solutions in preparation of a drug for promoting immunity, preventing and/or treating solid tumors (especially malignant solid tumors) and hematologic tumors, and/or expanding CD8⁺ T cells.

In some preferred embodiments of the present invention, the solid tumors refer to bladder cancer, bone cancer, brain cancer, breast cancer, colorectal cancer, esophageal cancer, ocular cancer, head and neck cancer, kidney cancer, lung cancer, melanoma, ovarian cancer, pancreatic cancer or prostate cancer.

In some preferred embodiments of the present invention, the hematologic tumors refer to chronic lymphocytic leukemia (CLL), small lymphocytic lymphoma (SLL), follicular lymphoma (FL), diffuse large B-cell lymphoma (DLBCL), mantle cell lymphoma (MCL), Waldenstrom's macroglobulinemia, multiple myeloma, extranodal marginal zone B-cell lymphoma, nodal marginal zone B-cell lymphoma, Burkitt lymphoma, non-Burkitt high-grade B-cell lymphoma, primary mediastinal B-cell lymphoma (PMBL), immunoblastic large cell lymphoma, precursor B-cell lymphoblastic lymphoma, B-cell prolymphocytic leukemia, lymphplasmacytic lymphoma, splenic marginal zone lymphoma, plasma cell myeloma, plasmacytoma, mediastinal (thymic) large B-cell lymphoma, intravascular large B-cell lymphoma, primary effusion lymphoma, or lymphomatoid granulomatosis.

In a third aspect, the present invention also provides a kit, which includes any one of the above-mentioned human interleukin 2-polyethylene glycol conjugate.

In a fourth aspect, the present invention also provides a method for preventing and/or treating solid tumors (especially malignant solid tumors) and hematologic tumors, comprising the step of administering a therapeutically effective amount of any one of the above-mentioned human interleukin 2-polyethylene glycol conjugate to a patient in need.

In some preferred embodiments of the present invention, the solid tumors refer to bladder cancer, bone cancer, brain cancer, breast cancer, colorectal cancer, esophageal cancer, ocular cancer, head and neck cancer, kidney cancer, lung cancer, melanoma, ovarian cancer, pancreatic cancer or prostate cancer.

In some preferred embodiments of the present invention, the hematologic tumors refer to chronic lymphocytic leukemia (CLL), small lymphocytic lymphoma (SLL), follicular lymphoma (FL), diffuse large B-cell lymphoma (DLBCL), mantle cell lymphoma (MCL), Waldenstrom's macroglobulinemia, multiple myeloma, extranodal marginal zone B-cell lymphoma, nodal marginal zone B-cell lymphoma, Burkitt lymphoma, non-Burkitt high-grade B-cell lymphoma, primary mediastinal B-cell lymphoma (PMBL), immunoblastic large cell lymphoma, precursor B-cell lymphoblastic lymphoma, B-cell prolymphocytic leukemia, lymphplasmacytic lymphoma, splenic marginal zone lymphoma, plasma cell myeloma, plasmacytoma, mediastinal (thymic) large B-cell lymphoma, intravascular large B-cell lymphoma, primary effusion lymphoma, or lymphomatoid granulomatosis.

In some preferred embodiments of the present invention, the human interleukin 2-polyethylene glycol conjugate may be administered alone or in combination with one or more other anti-tumor drugs.

In a fifth aspect, the present invention also provides a method for promoting immune function, comprising the step of administering a therapeutically effective amount of any one of the above-mentioned human interleukin 2-polyethylene glycol conjugate to a patient in need.

In some preferred embodiments of the present invention, the promoting immune function is to promote the immune function of the human interleukin-2.

In a sixth aspect, the present invention also provides a method for expanding CD8⁺ T cells, comprising the step of administering a therapeutically effective amount of any one of the above-mentioned human interleukin 2-polyethylene glycol conjugate to a patient in need.

The technical solutions provided by the present invention have the following advantages:
(1) The novel unnatural amino acid of the present invention can be introduced to a designated site through the codon expansion technology, so as to realize the accurate site-directed coupling of the PEG and the interleukin-2, and overcome the defects that the traditional random coupling method cannot accurately couple, and has high product homogeneity.
(2) The unnatural amino acid of the present invention is a lysine analogue containing a terminal carbonyl and aryl in the structure thereof, compared with the common lysine analogue containing an azide group (for example, Lys-azido), the unnatural amino acid is simpler to prepare, safer, less likely to be inactivated when being inserted into the protein, higher in binding rate with the PEG, and better in stability of the obtained conjugate, and the coupling efficiency can still exceed 95% after denaturation and renaturation of the recombinant protein inclusion body.
(3) Through the design and screening of mutation sites, the present invention obtains the interleukin-2 mutation site which can reduce the binding activity of IL-2Rα and keep the binding activity of IL-2Rβ and IL-2Rγ relatively unchanged, so that the site-directed modified human interleukin 2-polyethylene glycol conjugate can specifically promote the proliferation of CD8⁺ T cells in the tumor microenvironment, but has no obvious effect on the proliferation of CD4⁺ T cells, thereby being beneficial to the immunotherapy of tumors.
(4) The conjugate of the present invention realizes the extension of the half-life of IL-2 in vivo through PEG coupling, thereby reducing the administration frequency of patients.

### DESCRIPTION OF THE DRAWINGS

FIG. 1 is a schematic diagram of expression plasmid NB1S3-WT.
FIG. 2 is a schematic diagram of helper plasmid NB1W.

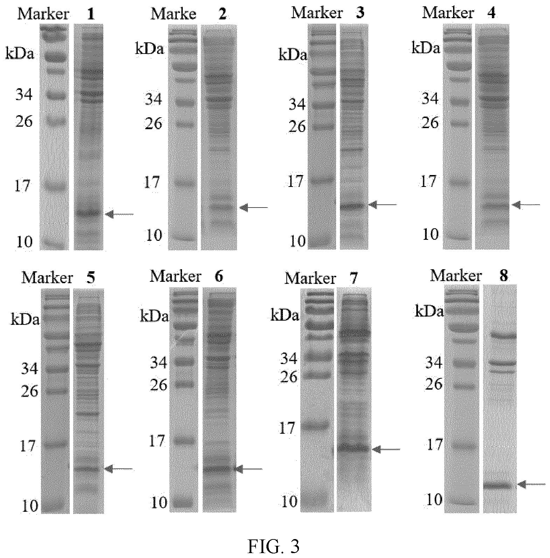

FIG. 3 is an SDS-PAGE electrophoretogram of fermentation products obtained by rhIL-2 expression strains with different mutation sites obtained in Example 2 after adding an unnatural amino acid NBOK (the arrows indicate bands position of target products); wherein Lane 1: rhIL2-K35-BL21 strains fed with NBOK are lysed and precipitated after centrifugation; Lane 2: rhIL2-T41-BL21 strains fed with NBOK are lysed and precipitated after centrifugation; Lane 3: rhIL2-K43-BL21 strains fed with NBOK are lysed and precipitated after centrifugation; Lane 4: rhIL2-Y45-BL21 strains fed with NBOK are lysed and precipitated after centrifugation; Lane 5: rhIL2-E61-BL21 strains fed with NBOK are lysed and precipitated after centrifugation; Lane 6: rhIL2-K64-BL21 strains fed with NBOK are lysed and precipitated after centrifugation; Lane 7: rhIL2-P65-BL21 strains fed with NBOK are lysed and precipitated after centrifugation; and Lane 8: rhIL2-Y45-BL21 strains fed with NBOK are precipitated and washed to obtain inclusion bodies.
FIG. 4A and FIG. 4B are RP-HPLC profiles of mutant rhIL-2 before and after coupling with PEG in Example 3 respectively; wherein, FIG. 4A shows each mutant rhIL-2 before coupling, wherein the main peak of the target protein is at about 22.5 min; and FIG. 4B shows each mutant rhIL-2 (PEG is 30 KD PEG) after coupling, wherein the main peak of the target protein is at about 21 min.
FIG. 5 is an RP-HPLC profile of the conjugate 30 KD PEG-rhIL2-Y45 in Example 4 before and after column chromatography.
FIG. 6 is a mass spectrogram of rhGH-V91 in Example 9.
FIG. 7 is an SDS-PAGE electrophoregram of recombinant IL-2 strains containing different unnatural amino acids obtained in Example 10.
FIG. 8 is an SDS-PAGE electrophoregram of mutant IL-2 crude proteins containing different unnatural amino acids obtained in Example 10.
FIG. 9A and FIG. 9B are RP-HPLC profiles of mutant rhIL-2 (rhIL2-T41NPAK) before and after coupling with PEG in Example 11 respectively; wherein, FIG. 9A shows the mutant rhIL-2 before coupling, wherein the main peak of the target protein is at about 22.380 min; and FIG. 9B shows the mutant rhIL-2 (PEG is 30 KD PEG) after coupling, wherein the main peak of the target protein is at about 21.175 min.
FIG. 10 is an RP-HPLC profile of the conjugate after purification in Example 12.
FIG. 11A shows western blot experimental results of pSTAT5 and β-actin protein of CTLL2 cells stimulated by reference rhIL2 with different concentrations at 37°C for 10 minutes, and an EC50 curve fitted according to gray scale results in Example 13.
FIG. 11B shows western blot experimental results of pSTAT5 and β-actin protein of CTLL2 cells stimulated by T41NPAK mutated IL-2 (i.e., rhIL2-T41NPAK) and 30 KD PEG-rhIL2-T41NPAK with different concentrations at 37°C for 10 minutes, and an EC50 curve fitted according to the gray scale results in Example 13.
FIG. 11C shows western blot experimental results of pSTAT5 and β-actin protein of YT cells stimulated by reference rhIL2 with different concentrations at 37°C for 10 minutes, and an EC50 curve fitted according to gray scale results in Example 13.
FIG. 11D shows western blot experimental results of pSTAT5 and β-actin protein of YT cells stimulated by T41NPAK mutated IL-2 (i.e., rhIL2-T41NPAK) and 30 KD PEG-rhIL2-T41NPAK with different concentrations at 37°C for 10 minutes, and an EC50 curve fitted according to the gray scale results in Example 13.

### EMBODIMENT

The technical solutions of the present invention will be further described in detail hereinafter with reference to the specific examples.

Unless otherwise specified, the reagents or raw materials used in the preparation examples and examples of the present invention are all commercially available products; and the experimental methods used are all conventional methods in this field unless otherwise specified.

Human YT cells are disclosed in the literature "Yodoi, J. et al. (1985). TCGF (IL 2)-receptor inducing factor(s). I. Regulation of IL 2 receptor on a natural killer-like cell line (YT cells).Journal of Immunology, 134(3), 1623-1630", and can be obtained by the public from Novocodex Biopharmaceuticals Co., Ltd.

### Preparation Example 1 Preparation of unnatural amino acid NBOK

The structural formula of NBOK was as follows:

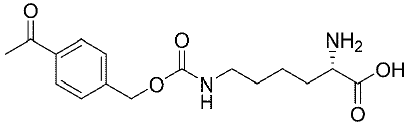

The reaction process was shown in the following:

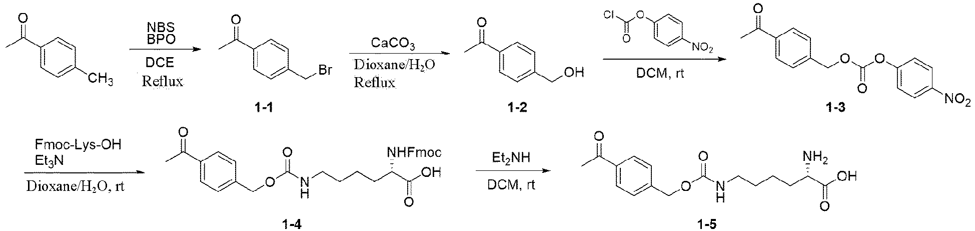

The preparation process included the following steps.
a) *P*-methyl acetophenone (4.0 mL, 30.0 mmol), a solvent DCE (50.0 mL), NBS (6.41 g, 36.0 mmol) and BPO (0.05 g, 0.3 mmol) were added into a reaction flask. The mixture was refluxed at 80°C for 24 hours, then the container was cooled in ice water to precipitate a solid, and the solid was removed by filtration. The solution was washed with saturated Na₂CO₃ for 3 times, and then extracted with DCM for 3 times. Organic phases were combined, dried with anhydrous sodium sulfate, filtered, and concentrated under a reduced pressure to obtain a crude product **1-1** (5.46 g, yield 85%), which was directly used in next step without purification.
b) The product **1-1** (2.73 g, 12.80 mmol), a solvent dioxane (40 mL) and water (40 mL) were added into a reaction flask, and then added with calcium carbonate (7.68 g, 76.8 mmol). The mixture was refluxed at 105°C for 24 hours, cooled to room temperature, filtered to remove the solid, and then extracted with DCM for 3 times. Organic phases were combined, concentrated under a reduced pressure, and purified by column chromatography (eluent: PE:EA = 3:1) to obtain a product **1-2** (1.80 g, yield 94%).
c) *P*-nitrophenyl chloroformate (2.90 g, 14.4 mmol) and a solvent DCM (10.0 mL) were added into a two-neck reaction flask, cooled to 0°C, added with the product **1-2** (1.80 g, 12.0 mmol) and pyridine (1.2 mL, 14.4 mmol), and stirred at room temperature for 18 hours, and then the reaction solution was added with a saturated sodium carbonate solution (10 mL), and extracted with DCM (50 mL) for 3 times. Organic phases were combined, washed with water twice, dried with anhydrous sodium sulfate, filtered, concentrated under a reduced pressure, and purified by column chromatography (eluent: PE:EA = 5:1) to obtain a product **1-3** (3.14 g, yield 83%).
d) The product **1-3** (1.26 g, 4.0 mmol) and Fmoc-Lys-OH hydrochloride (1.40 g, 3.33 mmol), a solvent dioxane (15 mL) and water (5 mL) were added into a reaction flask, and then added with triethylamine (1.2 mL, 8.3 mmol). The mixture was reacted at room temperature for 24 hours, and then was added with an appropriate amount of 1 M HCl solution, extracted with DCM, and concentrated under a reduced pressure to obtain a crude product **1-4**, which was directly used in next step.
e) The product **1-4** (1.10 g, 0.19 mmol) was dissolved in DCM (10 mL) in a reaction flask, and added with diethylamine (5.0 mL) to react at room temperature for 6 hours to precipitate a product. The product was filtered, and then pulped with DCM for 3 times to obtain the target product **1-5** (817 mg, two-step yield 63%).

¹H-NMR (400 MHz, heavy water) δ 8.04 (d, *J =* 8.4 Hz, 2H), 7.55 (d, *J =* 8.0 Hz, 2H), 5.21 (s, 2H), 3.74 (t, *J =* 6.0 Hz, 1H), 3.17 (t, *J =* 6.4 Hz, 2H), 2.70 (s, 3H), 1.95 - 1.83 (m, 2H), 1.62 - 1.52 (m, 2H), 1.47 - 1.35 (m, 2H).

### Preparation Example 2 Preparation of unnatural amino acid NPAK

The structural formula of NPAK was as follows:

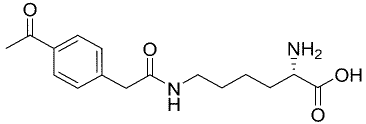

The reaction process was shown in the following:

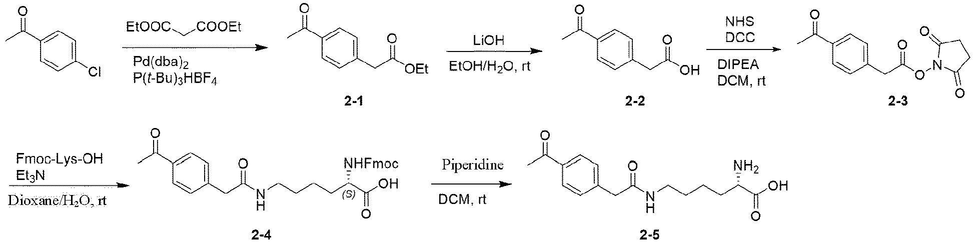

The preparation process included the following steps.
a) *P*-chloroacetophenone (1.00 g, 6.47 mmol) was added into a reaction flask, diethyl malonate (6.84 g, 47.70 mmol), KHCO₃ (0.97 g, 9.70 mmol) and K₂CO₃ (1.34 g, 9.70 mmol) were added under a nitrogen atmosphere, and then Pd(dba)₂ (0.019 g, 0.030 mmol) and P(*t*-Bu)₃HBF₄ (0.021 g, 0.071 mmol) were added. Nitrogen was replaced for protection after addition, and the mixture was heated to 160°C to react for 40 hours. After the completion of reaction was detected by TLC, the reaction solution was added with water (30 mL), extracted with EA for 3 times. Organic phases were combined, washed with water twice, dried with anhydrous sodium sulfate, filtered, and concentrated under a reduced pressure at 0°C to 5°C to obtain a colorless and transparent liquid, and the liquid was purified by column chromatography (eluent: PE:EA= 10:1) to obtain a product **2-1** (0.80 g, yield 60%).
b) LiOH (0.30 g, 11.64 mmol) and water (5.0 mL), ethanol (10 mL), and the product **2-1** (0.80 g, 3.88 mmol) were added into a reaction flask, and stirred at room temperature for 2 hours. After the completion of reaction was detected by TLC, the reaction solution was added with 2 M HCl solution to adjust the pH value to be 1 to 2, and then extracted with EA for 3 times. Organic phases were combined, washed with water twice, dried with anhydrous sodium sulfate, filtered, and concentrated under a reduced pressure to obtain a product **2-2** (0.5 g, yield 72%).
c) The product **2-2** (0.20 g, 1.12 mmol) was added into a reaction flask, and N-hydroxysuccinimide (NHS, 0.19 g, 1.68 mmol), DIPEA (0.07 g, 0.56 mmol) and DCM (2.0 mL) were added in sequence. The mixture was cooled to 0°C to 5°C, added with a solution of DCC (0.23 g, 1.12 mmol) and DCM (2.0 mL) to react in a thermally insulated manner for 2 hours, and heated to room temperature to stir overnight. After the completion of reaction was detected by TLC, the reaction solution was filtered, and washed with DCM, and the mother solution was concentrated under a reduced pressure, and purified by column chromatography (eluent: PE:EA = 5:1) to obtain a product **2-3** (0.19 g, yield 62%).
d) The product **2-3** (0.10 g, 0.36 mmol) was added into a reaction flask, and triethylamine (0.04 g, 0.36 mmol), Fmoc-Lys-OH hydrochloride (0.13 g, 0.36 mmol), dioxane (2.0 mL) and water (2.0 mL) were added in sequence. The mixture was stirred at room temperature to react for 18 hours. After the completion of reaction was detected by TLC, the reaction solution was concentrated under a reduced pressure, extracted with EA for 3 times, dried with anhydrous sodium sulfate, concentrated under a reduced pressure, and purified by column chromatography (eluent: DCM: MeOH = 15:1) to obtain a oily liquid product **2-4** (0.03 g, yield 61%).
e) The product **2-4** (0.08 g, 0.15 mmol), DCM (1.0 mL) and piperidine (0.04 g, 0.47 mmol) were added into a reaction flask, and stirred at room temperature for 3 hours. After the completion of reaction was detected by TLC, the reaction solution was concentrated under a reduced pressure, pulped with petroleum ether (5 mL) for 1 hour, and filtered. The filter cake was pulped with petroleum ether (5 mL) for 1 hour again, and filtered, and then the filter cake was repeatedly pulped with ethanol for 4 times to remove residual piperidine to finally obtain a white-like solid **2-5** (0.02 g, yield 43%).

¹H-NMR (400 MHz, heavy water) δ 7.85 (d, *J =* 8.2 Hz, 2H), 7.33 (d, *J =* 8.2 Hz, 2H), 3.94 (t, *J =* 6.3 Hz, 1H), 3.56 (s, 2H), 3.12 (t, *J =* 6.8 Hz, 2H), 2.54 (s, 3H), 1.80 - 1.70 (m, 2H), 1.54 - 1.45 (m, 2H), 1.40 - 1.224 (m, 2H).

### Preparation Example 3 Preparation of unnatural amino acid NBPK

The structural formula of NBPK was as follows:

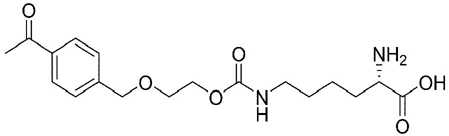

The reaction process was shown in the following:

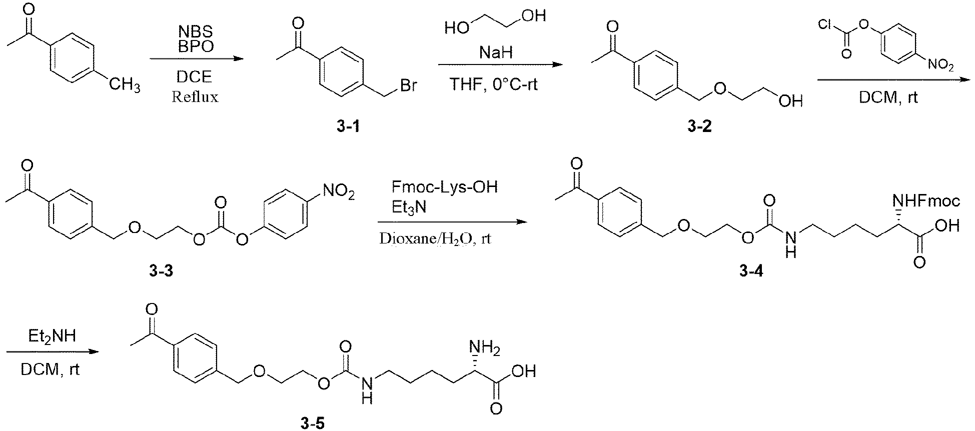

The preparation process included the following steps.
a) *P*-methyl acetophenone (4.0 mL, 30.0 mmol), a solvent DCE (50.0 mL), and NBS (6.41 g, 36.0 mmol) and BPO (0.05 g, 0.3 mmol) were added into a reaction flask, and the mixture was refluxed at 80°C for 24 hours. After the completion of reaction was detected by TLC, the container was cooled in ice water to precipitate a solid, and the solid was removed by filtration. The solution was washed with saturated Na₂CO₃ for 3 times, and extracted with DCM for 3 times. Organic phases were combined, added with anhydrous sodium sulfate for drying, filtered, and concentrated under a reduced pressure to obtain a crude product **3-1** (5.46 g, yield 85%), which was directly used in next step without purification.
b) NaH (0.58 g, 14.64 mmol, 60%) and a dry solvent THF (20 mL) were added into a reaction flask, and ethylene glycol (6.7 mL, 122.0 mmol) was slowly added under cooling in an ice bath. The mixture was stirred at room temperature for 1 hour. Subsequently, the product **3-1** (2.60 g, 12.2 mmol) was added, and heated and refluxed at 70°C for 48 hours until the reaction was completed. Saturated NH₄Cl was slowly and dropwise added under cooling in an ice bath to quench NaH, and then the solution was washed with water, and extracted with EtOAc for 3 times. Organic phases were combined, dried with anhydrous sodium sulfate, filtered, concentrated under a reduced pressure, and purified by column chromatography (eluent: PE:EA = 2:1) to obtain a product **3-2** (1.39 g, yield 59%).
c) The product **3-2** (1.39 g, 7.2 mmol) and a solvent DCM (10 mL) were added into a reaction flask, and *p*-nitrophenyl chloroformate (1.74 g, 8.64 mmol) and pyridine (0.7 mL, 8.64 mmol) were added under cooling in an ice bath. The mixture was stirred at room temperature for 18 hours. After the completion of reaction was detected by TLC, the reaction solution was added with water for washing, and extracted with EtOAc for 3 times. Organic phases were combined, dried with anhydrous sodium sulfate, filtered, concentrated under a reduced pressure, and then purified by column chromatography (eluent: PE:EA = 3:1) to obtain a product **3-3** (2.27 g, yield 88%).
d) The product **3-3** (2.27 g, 6.32 mmol), a solvent dioxane (16 mL) and water (4 mL), and Fmoc-Lys-OH hydrochloride (2.13 g, 5.27 mmol) were added into a reaction flask, and then triethylamine (1.85 mL, 13.2 mmol) was added. The mixture was stirred at room temperature for 18 hours until the reaction was completed. The solution was added with an appropriate amount of 1 M HCl to adjust the pH value to be about 2, and extracted with ethyl acetate. Organic phases were combined, dried with anhydrous sodium sulfate, filtered, and concentrated under a reduced pressure to obtain a crude product **3-4**, which was directly used in next step.
e) The product **3-4** in the previous step was dissolved in DCM (10 mL) in a reaction flask, and added with diethylamine (5 mL) to react at room temperature for 6 hours. After the completion of reaction was detected by TLC, the reaction solution was concentrated under a reduced pressure, and purified by column chromatography (eluent: DCM: MeOH: H₂O = 40:10:1) to obtain a white solid product **3-5** (0.95 g, two-step yield 49%).

¹H-NMR (400 MHz, heavy water) δ 8.04 (d, *J =* 8.0 Hz, 2H), 7.56 (d, *J =* 8.0 Hz, 2H), 4.72 (s, 2H), 4.25 (s, 2H), 3.81 (s, 2H), 3.74 (t, *J =* 6.0 Hz, 1H), 3.16 - 3.08 (m, 2H), 2.70 (s, 3H), 1.97 - 1.79 (m, 2H), 1.60 - 1.48 (m, 2H), 1.48 - 1.35 (m, 2H).

### Preparation Example 4 Preparation of unnatural amino acid NPOK

The structural formula of NPOK was as follows:

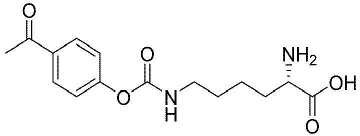

The reaction process was shown in the following:

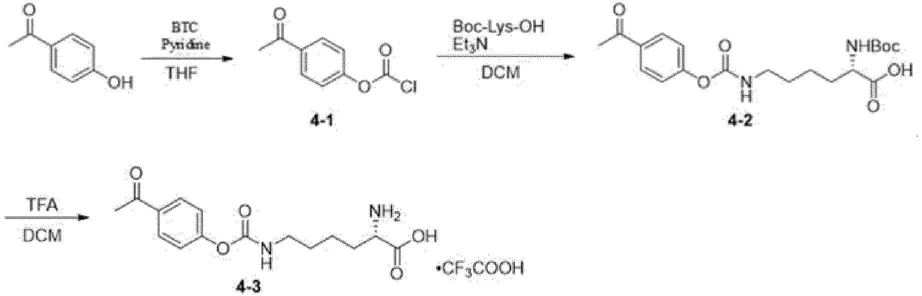

The preparation process included the following steps.
a) Triphosgene (BTC, 2.18 g, 7.35 mmol) and a solvent THF (10.0 mL) were added into a reaction flask, and *p*-hydroxyacetophenone (2.0 g, 14.7 mmol) and pyridine (1.5 mL, 17.64 mmol) were added under cooling in an ice bath. The mixture was reacted at room temperature for 24 hours. After the completion of reaction was detected by TLC, the reaction solution was added with an appropriate amount of water, and extracted with EtOAc for 3 times. Organic phases were combined, dried with anhydrous sodium sulfate, filtered, and concentrated under a reduced pressure to obtain a crude product **4-1** (1.20 g), which was directly used in next step.
b) Boc-lysine (1.1 g, 5.0 mmol), DCM (10.0 mL), and the product **4-1** (1.20 g) and triethylamine (2 mL, 15 mmol) were added into a reaction flask. The mixture was stirred at room temperature for 24 hours. After the completion of reaction was detected by TLC, the reaction solution was added with an appropriate amount of 1 M HCl to adjust pH to be faintly acid, and extracted with DCM for 3 times. Organic phases were combined, dried with anhydrous sodium sulfate, filtered, concentrated under a reduced pressure, and purified by column chromatography (eluent: DCM:MeOH = 5:1) to obtain a product **4-2** (1.70 g, yield 84%).
c) The product **4-2** (1.70 g, 4.2 mmol), a solvent DCM (5 mL), and a trifluoroacetic acid (5 mL) were added into a reaction flask. The mixture was reacted at room temperature for 1 hour. After the completion of reaction was detected by TLC, the reaction solution was directly concentrated under a reduced pressure, and purified by column chromatography (eluent: DCM: MeOH: H₂O = 40:10:1) to obtain a product **4-3** (1.19 g, yield 67%).

¹H-NMR (400 MHz, heavy water) δ 8.09 (d, *J =* 8.6 Hz, 2H), 7.31 (d, *J =* 8.6 Hz, 2H), 3.78 (t, *J =* 6.0 Hz, 1H), 3.27 (t, *J =* 6.8 Hz, 2H), 2.70 (s, 3H), 1.98 - 1.87 (m, 2H), 1.72 - 1.60 (m, 2H), 1.55 - 1.43 (m, 2H).

### Preparation Example 5 Preparation of unnatural amino acid NBGK

The structural formula of NBGK was as follows:

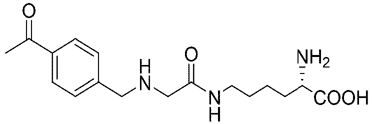

The reaction process was shown in the following:

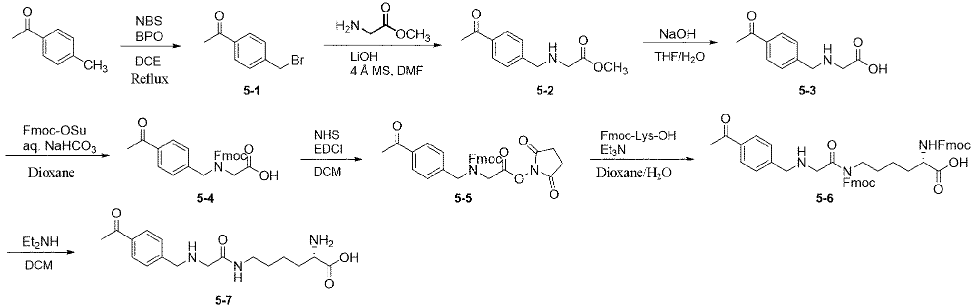

The preparation process included the following steps.
a) *P*-methyl acetophenone (8.0 mL, 60.0 mmol), a solvent DCE (80.0 mL), and NBS (12.82 g, 72.0 mmol) and BPO (145 mg, 0.6 mmol) were added into a reaction flask, and the mixture was refluxed at 90°C for 24 hours. After the completion of reaction was detected by TLC, the container was cooled in ice water to precipitate a solid, and the solid was removed by filtration. The solution was washed with saturated Na₂CO₃ for 3 times, and extracted with DCM for 3 times. Organic phases were combined, dried with anhydrous sodium sulfate, filtered, and concentrated under a reduced pressure to obtain a crude product **5-1** (11.12 g, yield 87%), which was directly used in next step without purification.
b) 4 Å MS (14 g) and LiOH (1.45 g, 34.54 mmol) were dissolved in DMF (70 mL) in a reaction flask and stirred at room temperature for 20 minutes, the added with glycinemethylester hydrochloride (2.0 g, 15.7 mmol) and stirred for 45 minutes, and then added with the product **5-1** (4.0 g, 18.8 mmol) and stirred at room temperature for 18 hours. After the completion of reaction was detected by TLC, the solid was removed by filtration, the filter cake was washed with EA, and the filtrate was washed with water twice, dried with anhydrous sodium sulfate, and concentrated under a reduced pressure to obtain a crude product **5-2**, which was directly used in next step.
c) The product **5-2** in the previous step was dissolved in dioxane (20 mL) in a reaction flask, and slowly and dropwise added with 1 M NaOH to react for 2 hours, and then the completion of hydrolysis reaction was monitored by TLC to obtain a product **5-3.** The product **5-3** was added with 20 mL of saturated NaHCO₃, slowly added with Fmoc-OSu dissolved in dioxane (10 mL), and stirred at room temperature overnight. After the completion of reaction was monitored by TLC, the reaction solution was adjusted to be faintly acid with 1 M HCl, extracted with EA, dried with anhydrous sodium sulfate, concentrated under a reduced pressure, and purified by column chromatography (eluent: DCM:MeOH = 10:1) to obtain a product **5-4** (3.60 g, yield 89%).
d) The product **5-3** (3.60 g, 8.0 mmol), NBS (1.10 g, 9.6 mmol) and EDCI (1.85 g, 9.6 mmol), and a solvent DCM (50 mL) were added into a reaction flask. The mixture was reacted at room temperature for 18 hours. After the completion of reaction was monitored by TLC, the reaction solution was washed with water for 3 times, dried with anhydrous sodium sulfate, and concentrated under a reduced pressure to obtain a product **5-5** (3.20 g, yield 75%).
e) The product **5-5** (3.20 g, 6.0 mmol), a solvent dioxane (40 mL) and water (10 mL), and Fmoc-Lys-OH hydrochloride (3.0 g, 7.2 mmol) were added into a reaction flask, and then triethylamine (2.0 mL, 15.0 mmol) was added. The mixture was stirred at room temperature for 18 hours until the reaction was completed. The solution was added with an appropriate amount of 1 M HCl to adjust the pH value to be about 2, and extracted with EA. Organic phases were combined, dried with anhydrous sodium sulfate, filtered, concentrated under a reduced pressure, and purified by column chromatography (eluent: DCM: MeOH: AcOH = 20:1:0.5) to obtain a product **5-5** (3.50 g, yield 75%).
f) The product **5-5** was dissolved in DCM (20 mL) in a reaction flask, and added with diethylamine (20 mL) to react at room temperature for 6 hours. After the completion of reaction was monitored by TLC, the reaction solution was concentrated under a reduced pressure, and purified by column chromatography (eluent: DCM: MeOH: H₂O=30:10:1) to obtain a final product **5-7** of white powder (0.55 g, yield 37%).

¹H-NMR (400 MHz, heavy water) δ 7.98 (d, *J =* 8.2 Hz, 2H), 7.50 (d, *J =* 8.2 Hz, 2H), 3.84 (s, 2H), 3.71 (s, 1H), 3.31 (s, 2H), 3.17 (t, *J* = 6.9 Hz, 2H), 2.67 (s, 3H), 1.97 - 1.73 (m, 2H), 1.58 - 1.45 (m, 2H), 1.44- 1.27 (m, 2H).

### Preparation Example 6 Preparation of unnatural amino acid NPOK-2

The structural formula of NPOK-2 was as follows:

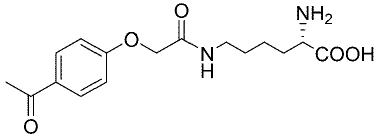

The reaction process was shown in the following:

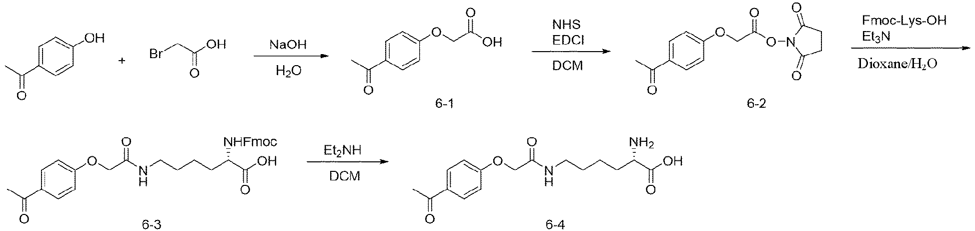

The preparation process included the following steps.
a) *P*-acetylphenol (2.05 g, 15.0 mmol) and a bromoacetic acid (2.50 g, 18.0 mmol) were added into a reaction flask, and then an aqueous solution (6 mL) of NaOH (1.20 g, 30 mmol) was added. The mixture was refluxed at 100°C for 24 hours until the reaction was completed. The reaction solution was cooled to room temperature, adjusted to be acidic by adding 1 M hydrochloric acid to precipitate a solid, and filtered to obtain a white crude product **6-1** (3.32 g, yield 113%), which was directly used in next step without purification.
b) The crude product **6-1** (3.32 g, 17.0 mmol) in the previous step was dissolved in DCM (50 mL) in a reaction flask, and added with NHS (2.35 g, 20.4 mmol) and EDCI (3.90 g, 20.4 mmol). The mixture was stirred at room temperature for 18 hours until the reaction was completed. The reaction solution was extracted with DCM. Organic phases were combined, dried with anhydrous sodium sulfate, filtered, concentrated under a reduced pressure, and purified by column chromatography (eluent: DCM: MeOH: AcOH = 20:1:0.5) to obtain a product **6-2** (1.67 g, yield 38%).
c) The product **6-2** (1.67 g, 5.7 mmol), a solvent dioxane (20 mL) and water (50 mL), and Fmoc-Lys-OH hydrochloride (1.9 g, 4.8 mmol) were added into a reaction flask, and then triethylamine (1.7 mL, 12.0 mmol) was added. The mixture was stirred at room temperature for 18 hours until the reaction was completed. The solution was added with an appropriate amount of 1 M HCl to adjust the pH value to be about 2, and extracted with EA. Organic phases were combined, dried with anhydrous sodium sulfate, filtered, and concentrated under a reduced pressure. The crude product **6-3** obtained was directly dissolved in DCM (10 mL), and then added with diethylamine (5 mL). The mixture was stirred at room temperature for 18 hours until the reaction was completed. The reaction solution was concentrated under a reduced pressure, and purified by column chromatography (eluent: DCM: MeOH: H₂O = 40:10:1) to obtain a final product **6-4** (709 mg, two-step yield 39%).

¹H-NMR (400 MHz, heavy water) δ 7.91 (d, *J =* 8.8 Hz, 2H), 6.98 (d, *J =* 8.8 Hz, 2H), 4.60 (s, 2H), 3.59 (t, *J =* 6.4 Hz, 1H), 3.19 (t, *J =* 6.8 Hz, 2H), 2.52 (s, 3H), 1.87 - 1.65 (m, 2H), 1.56 - 1.40 (m, 2H), 1.35 - 1.15 (m, 2H).

### Preparation Example 7 Preparation of unnatural amino acid NBGK-2

The structural formula of NBGK-2 was as follows:

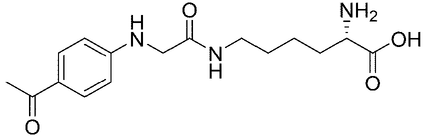

The reaction process was shown in the following:

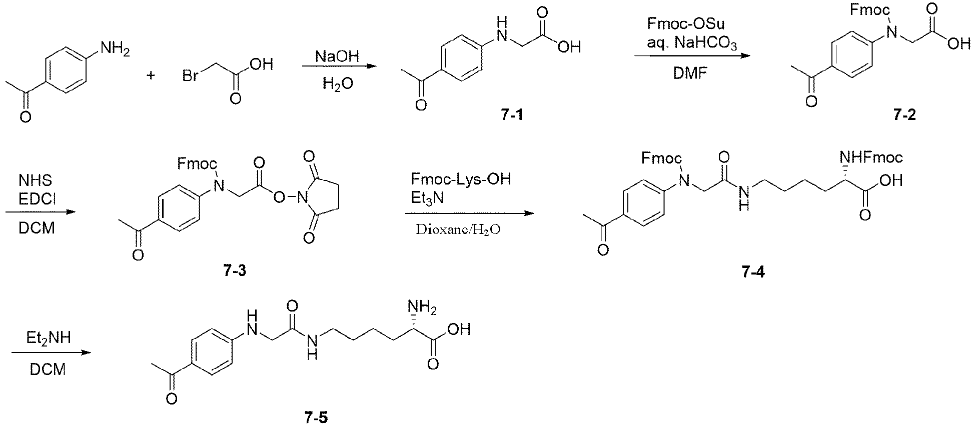

The preparation process included the following steps.
a) A bromoacetic acid (2.10 g, 15.0 mmol) and an aqueous solution (10 mL) of NaOH (0.80 g, 20 mmol) were added into a reaction flask and stirred for 10 minutes, and then *p*-acetylaniline (1.40 g, 10.0 mmol) was added. The mixture was refluxed at 100°C for 18 hours until the reaction was completed. The reaction solution was cooled to room temperature, filtered, and washed with water to obtain a white crude product **7-1** (1.30 g, yield 67%), which was directly used in next step without purification.
b) The product **7-1** (1.30 g, 6.7 mmol) and an aqueous solution (20 mL) of NaHCO₃ (1.70 g, 20.1 mmol) were added into a reaction flask, and then Fmoc-OSu (2.80 g, 8.1 mmol) and DMF (20 mL) were added. The mixture was stirred at 60°C for 18 hours until the reaction was completed. The reaction solution was cooled to room temperature, and extracted with EA. The aqueous phase was retained and adjusted to have a pH value of about 2 with 1 M hydrochloric acid. Subsequently, the reaction solution was extracted with EA to obtain organic phases, which were dried with anhydrous sodium sulfate, filtered, and concentrated under a reduced pressure to obtain a product **7-2**, which was directly used in next step without purification.
c) The product **7-2** (about 6.7 mmol) in the previous step, NHS (0.90 g, 8.0 mmol) and EDCI (1.50 g, 8.0 mmol) were dissolved in DMF (50 mL) in a reaction flask. The reaction mixture was stirred at room temperature for 24 hours until the reaction was completed. The reaction solution was added with water, and extracted with DCM to obtain organic phase, which was dried with anhydrous sodium sulfate, filtered, and concentrated under a reduced pressure to obtain a product **7-3**, which was directly used in next step without purification.
d) The product **7-3** (about 6.7 mmol) in the previous step, Fmoc-Lys-OH hydrochloride (2.30 g, 5.6 mmol) and triethylamine (2.0 mL, 14.0 mmol) were added in to a reaction flask. The reaction mixture was stirred at room temperature for 3 hours until the reaction was completed. Subsequently, the reaction solution was adjusted to have a pH value of about 2 with 1 M hydrochloric acid, extracted with EA, dried with anhydrous sodium sulfate, filtered, and concentrated under a reduced pressure to obtain a product **7-4,** which was directly used in next step without purification.
e) The product **7-4** in the previous step, and a solvent DCM (20 mL) and diethylamine (10 mL) were added into a reaction flask. The reaction mixture was stirred at room temperature for 12 hours until the reaction was completed. The reaction solution was concentrated under a reduced pressure first, added with acetonitrile (50 mL) to dissolve again, and then concentrated under a reduced pressure, and the operation was repeated for 3 times to remove excess diethylamine. The reaction solution was added with DCM for pulping twice to obtain a final product **7-5** (1.65 g, total yield 51%).

¹H-NMR (400 MHz, heavy water) δ 7.71 (d, *J =* 8.8 Hz, 2H), 6.51 (d, *J =* 8.8 Hz, 2H), 3.80 (s, 2H), 3.53 (t, *J =* 6.8 Hz, 1H), 3.09 (t, *J =* 6.8 Hz, 2H), 2.39 (s, 3H), 1.73 - 1.60 (m, 2H), 1.42 - 1.33 (m, 2H), 1.25 - 1.14 (m, 2H).

### Example 1 Construction of expression strain expressing recombinant human IL-2 (rhIL-2) inserted with unnatural amino acid at directed site

### 1. Acquisition of expression plasmid NB1S3-WT of wild-type recombinant human IL-2

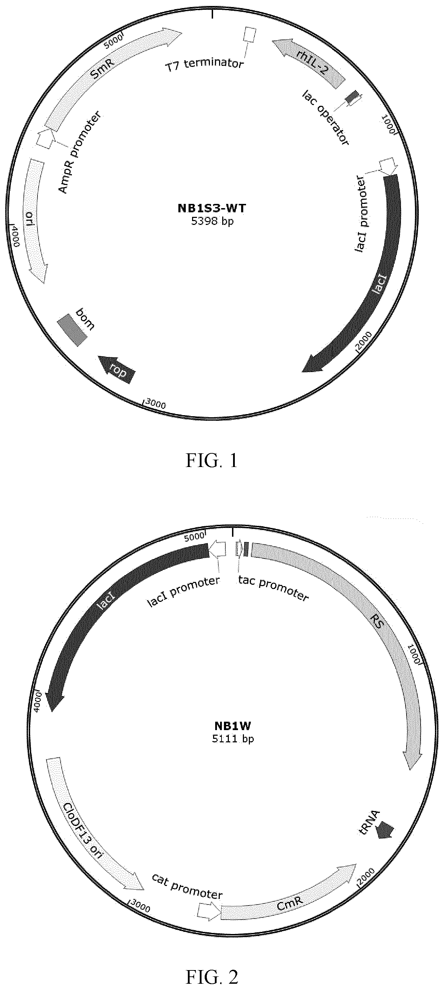

The precursor protein sequence (GenBank ID: CAA25292.1) of Homo sapiens IL-2 was obtained from the National Center for Biotechnology Information (NCBI), as shown in SEQ ID NO: 1. The N-terminal of the precursor sequence contained a signal peptide sequence consisting of 20 amino acids, which could be excised in the processing and maturing process of IL-2 protein molecules, so that a protein sequence (SEQ ID NO: 2) of mature Homo sapiens IL-2 was obtained after the signal peptide sequence was removed. According to the literature report (Liang S.M. et al. Journal of Biological Chemistry, 261(1): 334-337, 1986), the protein sequence of mature Homo sapiens IL-2 contained three Cysteines (Cys), wherein two Cysteines at the 58^{th} site and the 105^{th} site could form disulfide bonds, which were very important for the biological activity of Homo sapiens IL-2. The Cys at the 125^{th} site did not participate in the formation of disulfide bonds, but could interfere with the formation of normal disulfide bonds in the renaturation process of the protein inclusion body of recombinant Homo sapiens IL-2, so that the Cys at the 125^{th} site could be mutated into a serine (Ser), and the renaturation efficiency was improved without significantly affecting the activity. Meanwhile, in order to express a recombinant protein in *Escherichia coli,* it was necessary to add methionine (Met) to the N-terminal of the protein sequence for initiating the translation of the protein, so that a protein sequence (SEQ ID NO: 3) of mature recombinant human IL-2 was obtained. A gene sequence (SEQ ID NO: 4) encoding the recombinant human IL-2 was obtained by the reverse translation process of amino acids and codons and the optimization of the codons, and an encoding gene of the recombinant human IL-2 was obtained by whole gene synthesis. Then, the gene was linked to a NB1S3 expression vector by one-step sub-cloning (the expression vector was modified from a commercial vector pET-21a, and the screening marker of the ampicillin resistance gene was replaced by a spectinomycin resistance gene amplified from a commercial vector pCDF-duet1 by PCR), and an expression plasmid NB1S3-WT of wild-type recombinant human IL-2 was obtained (referring to FIG. 1), with a sequence shown in SEQ ID NO: 5.
SEQ ID NO: 1 
MYRMQLLSCIALSLALVTNSAPTSSSTKKTQLQLEHLLLDLQMILNGINNYKNPKLT
RMLTFKFYMPKKATELKHLQCLEEELKPLEEVLNLAQSKNFHLRPRDLISNINVIVLELKG
SETTFMCEYADETATIVEFLNRWITFCQSIISTLT
SEQ ID NO: 2
SEQ ID NO: 3 
MAPTSSSTKKTQLQLEHLLLDLQMILNGINNYKNPKLTRMLTFKFYMPKKATELKH
LQCLEEELKPLEEVLNLAQSKNFHLRPRDLISNINVIVLELKGSETTFMCEYADETATIVEF
LNRWITFSQSIISTLT
SEQ ID NO: 4 
ATGGCGCCTACATCCAGCTCGACCAAAAAGACGCAGCTGCAACTGGAACACCTG
CTCCTGGATCTGCAAATGATTCTTAACGGTATCAATAACTACAAAAATCCGAAACTGAC
CCGTATGCTGACGTTTAAATTCTATATGCCAAAGAAAGCGACCGAGCTGAAACATCTG
CAGTGCCTGGAAGAGGAACTGAAACCGCTGGAGGAAGTTTTGAACCTGGCTCAGTCT
AAAAACTTTCACCTGCGCCCTCGTGACCTGATTTCCAATATCAACGTGATTGTTCTGGA
ACTGAAAGGCTCTGAAACCACGTTTATGTGCGAGTACGCCGATGAAACCGCCACGAT
TGTGGAATTTCTGAATCGCTGGATCACCTTCTCCCAGAGCATTATTAGCACGCTGACCT
AA
SEQ ID NO: 5

GGCGAGAAAGGAAGGGAAGAAAGCGAAAGGAGCGGGCGCTAGGGCGCTGGCA

AGTGTAGCGGTCACGCTGCGCGTAACCACCACACCCGCCGCGCTTAATGCGCCGCTAC
AGGGCGCGTCCCATTCGCCAATCCGGATATAGTTCCTCCTTTCAGCAAAAAACCCCTC
AAGACCCGTTTAGAGGCCCCAAGGGGTTATGCTAGTTATTGCTCAGCGGTGGCAGCAG
CCAACTCAGCTTCCTTTCGGGCTTTGTTAGCAGCCGGATCTCAGTGGTGGTGGTGGTG
GTGCTCGAGTTAGGTCAGCGTGCTAATAATGCTCTGGGAGAAGGTGATCCAGCGATTC
AGAAATTCCACAATCGTGGCGGTTTCATCGGCGTACTCGCACATAAACGTGGTTTCAG
AGCCTTTCAGTTCCAGAACAATCACGTTGATATTGGAAATCAGGTCACGAGGGCGCAG
GTGAAAGTTTTAGACTGAGCCAGGTTCAAAACTTCCTCCAGCGGTTTCAGTTCCTCT
TCCAGGCACTGCAGATGTTTCAGCTCGGTCGCTTTCTTTGGCATATAGAATTTAAACGT
CAGCATACGGGTCAGTTTCGGATTTTGTAGTTATTGATACCGTTAAGAATCATTTGCA
GATCCAGGAGCAGGTGTTCCAGTTGCAGCTGCGTCTTTTTGGTCGAGCTGGATGTAGG
CGCCATATGTATATCTCCTTCTTAAAGTTAAACAAAATTATTTCTAGAGGGGAATTGTTA
TCCGCTCACAATTCCCCTATAGTGAGTCGTATTAATTTCGCGGGATCGAGATCTCGATC
CTCTACGCCGGACGCATCGTGGCCGGCATCACCGGCGCCACAGGTGCGGTTGCTGGC
GCCTATATCGCCGACATCACCGATGGGGAAGATCGGGCTCGCCACTTCGGGCTCATGA
GCGCTTGTTTCGGCGTGGGTATGGTGGCAGGCCCCGTGGCCGGGGGACTGTTGGGCG
CCATCTCCTTGCATGCACCATTCCTTGCGGCGGCGGTGCTCAACGGCCTCAACCTACT
ACTGGGCTGCTTCCTAATGCAGGAGTCGCATAAGGGAGAGCGTCGAGATCCCGGACA
CCATCGAATGGCGCAAAACCTTTCGCGGTATGGCATGATAGCGCCCGGAAGAGAGTC
AATTCAGGGTGGTGAATGTGAAACCAGTAACGTTATACGATGTCGCAGAGTATGCCGG
TGTCTCTTATCAGACCGTTTCCCGCGTGGTGAACCAGGCCAGCCACGTTTCTGCGAAA
CGCGGGAAAAAGTGGAAGCGGCGATGGCGGAGCTGAATTACATTCCCAACCGCGTGG
CACAACAACTGGCGGGCAAACAGTCGTTGCTGATTGGCGTTGCCACCTCCAGTCTGG
CCCTGCACGCGCCGTCGCAAATTGTCGCGGCGATTAAATCTCGCGCCGATCAACTGGG
TGCCAGCGTGGTGGTGTCGATGGTAGAACGAAGCGGCGTCGAAGCCTGTAAAGCGGC
GGTGCACAATCTTCTCGCGCAACGCGTCAGTGGGCTGATCATTAACTATCCGCTGGAT
GACCAGGATGCCATTGCTGTGGAAGCTGCCTGCACTAATGTTCCGGCGTTATTTCTTGA
TGTCTCTGACCAGACACCCATCAACAGTATTATTTTCTCCCATGAAGACGGTACGCGA
CTGGGCGTGGAGCATCTGGTCGCATTGGGTCACCAGCAAATCGCGCTGTTAGCGGGC
CCATTAAGTTCTGTCTCGGCGCGTCTGCGTCTGGCTGGCTGGCATAAATATCTCACTCG
CAATCAAATTCAGCCGATAGCGGAACGGGAAGGCGACTGGAGTGCCATGTCCGGTTT
TCAACAAACCATGCAAATGCTGAATGAGGGCATCGTTCCCACTGCGATGCTGGTTGCC
AACGATCAGATGGCGCTGGGCGCAATGCGCGCCATTACCGAGTCCGGGCTGCGCGTT
GGTGCGGATATCTCGGTAGTGGGATACGACGATACCGAAGACAGCTCATGTTATATCCC
GCCGTTAACCACCATCAAACAGGATTTTCGCCTGCTGGGGCAAACCAGCGTGGACCG
CTTGCTGCAACTCTCTCAGGGCCAGGCGGTGAAGGGCAATCAGCTGTTGCCCGTCTC

ACTGGTGAAAAGAAAAACCACCCTGGCGCCCAATACGCAAACCGCCTCTCCCCGCGC
GTTGGCCGATTCATTAATGCAGCTGGCACGACAGGTTTCCCGACTGGAAAGCGGGCA
GTGAGCGCAACGCAATTAATGTAAGTTAGCTCACTCATTAGGCACCGGGATCTCGACC
GATGCCCTTGAGAGCCTTCAACCCAGTCAGCTCCTTCCGGTGGGCGCGGGGCATGACT
ATCGTCGCCGCACTTATGAAGCTTCTATCGTCGCCGCACTTATGACTGTCTTCTTTATCA
TGCAACTCGTAGGACAGGTGCCGGCAGCGCTCTGGGTCATTTTCGGCGAGGACCGCT
TTCGCTGGAGCGCGACGATGATCGGCCTGTCGCTTGCGGTATTCGGAATCTTGCACGC
CCTCGCTCAAGCCTTCGTCACTGGTCCCGCCACCAAACGTTTCGGCGAGAAGCAGGC
CATTATCGCCGGCATGGCGGCCCCACGGGTGCGCATGATCGTGCTCCTGTCGTTGAGG
ACCCGGCTAGGCTGGCGGGGTTGCCTTACTGGTTAGCAGAATGAATCACCGATACGCG
AGCGAACGTGAAGCGACTGCTGCTGCAAAACGTCTGCGACCTGAGCAACAACATGA
ATGGTCTTCGGTTTCCGTGTTTCGTAAAGTCTGGAAACGCGGAAGTCAGCGCCCTGCA
CCATTATGTTCCGGATCTGCATCGCAGGATGCTGCTGGCTACCCTGTGGAACACCTACA
TCTGTATTAACGAAGCGCTGGCATTGACCCTGAGTGATTTTTCTCTGGTCCCGCCGCAT
CCATACCGCCAGTTGTTTACCCTCACAACGTTCCAGTAACCGGGCATGTTCATCATCAG
TAACCCGTATCGTGAGCATCCTCTCTCGTTTCATCGGTATCATTACCCCCATGAACAGA
AATCCCCCTTACACGGAGGCATCAGTGACCAAACAGGAAAAAACCGCCCTTAACATG
GCCCGCTTTATCAGAAGCCAGACATTAACGCTTCTGGAGAAACTCAACGAGCTGGAC
GCGGATGAACAGGCAGACATCTGTGAATCGCTTCACGACCACGCTGATGAGCTTTACC
GCAGCTGCCTCGCGCGTTTCGGTGATGACGGTGAAAACCTCTGACACATGCAGCTCC
CGGAGACGGTCACAGCTTGTCTGTAAGCGGATGCCGGGAGCAGACAAGCCCGTCAG
GGCGCGTCAGCGGGTGTTGGCGGGTGTCGGGGCGCAGCCATGACCCAGTCACGTAGC
GATAGCGGAGTGTATACTGGCTTAACTATGCGGCATCAGAGCAGATTGTACTGAGAGT
GCACCATATATGCGGTGTGAAATACCGCACAGATGCGTAAGGAGAAAATACCGCATCA
GGCGCTCTTCCGCTTCCTCGCTCACTGACTCGCTGCGCTCGGTCGTTCGGCTGCGGCG
AGCGGTATCAGCTCACTCAAAGGCGGTAATACGGTTATCCACAGAATCAGGGGATAAC
GCAGGAAAGAACATGTGAGCAAAAGGCCAGCAAAAGGCCAGGAACCGTAAAAAGG
CCGCGTTGCTGGCGTTTTTCCATAGGCTCCGCCCCCCTGACGAGCATCACAAAAATCG
ACGCTCAAGTCAGAGGTGGCGAAACCCGACAGGACTATAAAGATACCAGGCGTTTCC
CCCTGGAAGCTCCCTCGTGCGCTCTCCTGTTCCGACCCTGCCGCTTACCGGATACCTG
TCCGCCTTTCTCCCTTCGGGAAGCGTGGCGCTTTCTCATAGCTCACGCTGTAGGTATCT
CAGTTCGGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTAAGACAC
GACTTATCGCCACTGGCAGCAGCCACTGGTAACAGGATTAGCAGAGCGAGGTATGTAG
GCGGTGCTACAGAGTTCTTGAAGTGGTGGCCTAACTACGGCTACACTAGAAGGACAG
TATTTGGTATCTGCGCTCTGCTGAAGCCAGTTACCTTCGGAAAAAGAGTTGGTAGCTC

TTGATCCGGCAAACAAACCACCGCTGGTAGCGGTGGTTTTTTTGTTTGCAAGCAGCAG
ATTACGCGCAGAAAAAAAGGATCTCAAGAAGATCCTTTGATCTTTTCTACGGGGTCTG
ACGCTCAGTGGAACGAAAACTCACGTTAAGGGACTGCAGCTATTGTTTATTTTTCTA
AATACATTCAAATATGTATCCGCTCATGAGACAATAACCCTGATAAATGCTTCAATAATA
TTGAAAAAGGAAGAGTATGAGGGAAGCGGTGATCGCCGAAGTATCGACTCAACTATC
AGAGGTAGTTGGCGTCATCGAGCGCCATCTCGAACCGACGTTGCTGGCCGTACATTTG
TACGGCTCCGCAGTGGATGGCGGCCTGAAGCCACACAGTGATATTGATTTGCTGGTTA
CGGTGACCGTAAGGCTTGATGAAACAACGCGGCGAGCTTTGATCAACGACCTTTTGG
AAACTTCGGCTTCCCCTGGAGAGAGCGAGATTCTCCGCGCTGTAGAAGTCACCATTGT
TGTGCACGACGACATCATTCCGTGGCGTTATCCAGCTAAGCGCGAACTGCAATTTGGA
GAATGGCAGCGCAATGACATTCTTGCAGGTATCTTCGAGCCAGCCACGATCGACATTG
ATCTGGCTATCTTGCTGACAAAAGCAAGAGAACATAGCGTTGCCTTGGTAGGTCCAGC
GGCGGAGGAACTCTTTGATCCGGTTCCTGAACAGGATCTATTTGAGGCGCTAAATGAA
ACCTTAACGCTATGGAACTCGCCGCCCGACTGGGCTGGCGATGAGCGAAATGTAGTGC
TTACGTTGTCCCGCATTTGGTACAGCGCAGTAACCGGCAAAATCGCGCCGAAGGATGT
CGCTGCCGACTGGGCAATGGAGCGCCTGCCGGCCCAGTATCAGCCCGTCATACTTGAA
GCTAGACAGGCTTATCTTGGACAAGAAGATCGCTTGGCCTCGCGCGCAGATCAG
TTGGAAGAATTTGTCCACTACGTGAAAGGCGAGATCACCAAGGTAGTCGGCAAATAAT
GTCTAACAATTCGTTCAAGCCGAGGGGCCGCAAGATCCGGCCACGATGACCCGGTCG
TCGGTTCAGGGCAGGGTCGTTAAATAGCCGCTTATGTCTATTGCTGGTTTACCGGTTTA
TTGACTACCGGAAGCAGTGTGACCGT

### 2. Selection of site-directed mutation site

The P34 site, the K35 site, the T37 site, the R38 site, the L40 site, the T41 site, the F42 site, the K43 site, the F44 site, the Y45 site, the E61 site, the E62 site, the K64 site, the P65 site, the E67 site, the E68 site, the N71 site, the L72 site and the Y107 site of SEQ ID NO: 2 were selected as specific sites for site mutation, and this mutant IL-2 was used as a raw material and modified at a directed site.

### 3. Design of primer subjected to site-directed mutation and construction of mutant vector

For the P34 site, the K35 site, the T37 site, the R38 site, the L40 site, the T41 site, the F42 site, the K43 site, the F44 site, the Y45 site, the E61 site, the E62 site, the K64 site, the P65 site, the E67 site, the E68 site, the N71 site, the L72 site and the Y107 site of SEQ ID NO: 2, primers capable of mutating the codons encoding the amino acid into amber codons were respectively designed, and specific primers were shown in Table 1.

**Table 1 List of mutant primers**

| Mutation site and name | Mutation site sequence (5' → 3' direction) | Sequence number of corresponding sequence listing |
|---|---|---|
| P34-F | CAATAACTACAAAAATTAGAAACTGACC CGTATGC | SEQ ID NO: 6 |
| P34-R | GCATACGGGTCAGTTTCTAATTTTTGTAG TTATTG | SEQ ID NO: 7 |
| K35-F | TAACTACAAAAATCCGTAGCTGACCCGTA TGCTGA | SEQ ID NO: 8 |
| K35-R | TCAGCATACGGGTCAGCTACGGATTTTG TAGTTA | SEQ ID NO: 9 |
| T37-F | CAAAAATCCGAAACTGTAGCGTATGCTG ACGTTTA | SEQ ID NO: 10 |
| T37-R | TAAACGTCAGCATACGCTACAGTTTCGGA TTTTTG | SEQ ID NO: 11 |
| R38-F | AAATCCGAAACTGACCTAGATGCTGACG TTTAAAT | SEQ ID NO: 12 |
| R38-R | ATTTAAACGTCAGCATCTAGGTCAGTTTC GGATTT | SEQ ID NO: 13 |
| L40-F | GAAACTGACCCGTATGTAGACGTTTAAAT TCTATA | SEQ ID NO: 14 |
| L40-R | TATAGAATTTAAACGTCTACATACGGGTC AGTTTC | SEQ ID NO: 15 |
| T41-F | ACTGACCCGTATGCTGTAGTTTAAATTCT ATATGC | SEQ ID NO: 16 |
| T41-R | GCATATAGAATTTAAACTACAGCATACGG GTCAGT | SEQ ID NO: 17 |
| F42-F | GACCCGTATGCTGACGTAGAAATTCTATA TGCCAA | SEQ ID NO: 18 |
| F42-R | TTGGCATATAGAATTTCTACGTCAGCATA CGGGTC | SEQ ID NO: 19 |
| K43-F | CCGTATGCTGACGTTTTAGTTCTATATGCC AAAGA | SEQ ID NO: 20 |
| K43-R | TCTTTGGCATATAGAACTAAAACGTCAGC ATACGG | SEQ ID NO: 21 |
| F44-F | TATGCTGACGTTTAAATAGTATATGCCAA AGAAAG | SEQ ID NO: 22 |
| F44-R | CTTTCTTTGGCATATACTATTTAAACGTCA GCATA | SEQ ID NO: 23 |
| Y45-F | GCTGACGTTTAAATTCTAGATGCCAAAG AAAGCGA | SEQ ID NO: 24 |
| Y45-R | TCGCTTTCTTTGGCATCTAGAATTTAAAC GTCAGC | SEQ ID NO: 25 |
| E61-F | TCTGCAGTGCCTGGAATAGGAACTGAAA CCGCTGG | SEQ ID NO: 26 |
| E61-R | CCAGCGGTTTCAGTTCCTATTCCAGGCAC TGCAGA | SEQ ID NO: 27 |
| E62-F | GCAGTGCCTGGAAGAGTAGCTGAAACCG CTGGAGG | SEQ ID NO: 28 |
| E62-R | CCTCCAGCGGTTTCAGCTACTCTTCCAGG CACTGC | SEQ ID NO: 29 |
| K64-F | CCTGGAAGAGGAACTGTAGCCGCTGGAG GAAGTTT | SEQ ID NO: 30 |
| K64-R | AAACTTCCTCCAGCGGCTACAGTTCCTCT TCCAGG | SEQ ID NO: 31 |
| P65-F | GGAAGAGGAACTGAAATAGCTGGAGGA AGTTTTGA | SEQ ID NO: 32 |
| P65-R | TCAAAACTTCCTCCAGCTATTTCAGTTCC TCTTCC | SEQ ID NO: 33 |
| E67-F | GGAACTGAAACCGCTGTAGGAAGTTTTG AACCTGG | SEQ ID NO: 34 |
| E67-R | CCAGGTTCAAAACTTCCTACAGCGGTTT CAGTTCC | SEQ ID NO: 35 |
| E68-F | ACTGAAACCGCTGGAGTAGGTTTTGAAC CTGGCTC | SEQ ID NO: 36 |
| E68-R | GAGCCAGGTTCAAAACCTACTCCAGCGG TTTCAGT | SEQ ID NO: 37 |
| N71-F | GCTGGAGGAAGTTTTGTAGCTGGCTCAG TCTAAAA | SEQ ID NO: 38 |
| N71-R | TTTTAGACTGAGCCAGCTACAAAACTTC CTCCAGC | SEQ ID NO: 39 |
| L72-F | GGAGGAAGTTTTGAACTAGGCTCAGTCT AAAAACT | SEQ ID NO: 40 |
| L72-R | AGTTTTTAGACTGAGCCTAGTTCAAAAC TTCCTCC | SEQ ID NO: 41 |
| Y107-F | CACGTTTATGTGCGAGTAGGCCGATGAA ACCGCCA | SEQ ID NO: 42 |
| Y107-R | TGGCGGTTTCATCGGCCTACTCGCACATA AACGTG | SEQ ID NO: 43 |
| XbaI-F | TGAGCGGATAACAATTCCCCTCTAGAAA TAATTTTGTTTAAC | SEQ ID NO: 44 |
| XhoI-R | GATCTCAGTGGTGGTGGTGGTGGTGCTC GAG | SEQ ID NO: 45 |

The plasmid NB1S3-WT was subjected to double digestion with restriction endonucleases *Xba*I and *Xho*I to obtain a linearized DNA plasmid, which was used as a template. By using a high-fidelity DNA polymerase (purchased from Takara, catalog number R045A), the primer XbaI-F in Table 1 was paired with primers R of various sites, and the primer XhoI-R in Table 1 was paired with primers F of various sites. Mutant genes with amino acid codons at the K35 site, the T41 site, the K43 site, the Y45 site, the E61 site, the K64 site and the P65 site of IL-2 mutated into amber termination codons were obtained by PCR amplification and overlap PCR (for example, the linearized plasmid NB1S3-WT was used as a template, and XbaI-F and T41-R were used as a primer pair for PCR amplification to obtain an upstream fragment subjected to mutation at the T41 site; the linearized plasmid NB1S3-WT was used as a template, and XhoI-R and T41-F were used as a primer pair for PCR amplification to obtain a downstream fragment subjected to mutation at the T41 site; and then, the upstream fragment subjected to mutation at the T41 site and the downstream fragment subjected to mutation at the T41 site obtained above were used as templates, and XbaI-F and XhoI-R were used as a primer pair for overlap PCR amplification to obtain a full-length gene subjected to mutation at the T41 site). Then, a high-fidelity DNA cloning kit (purchased from NEB, catalog number E5520S) was used, and the fragment between two digestion sites *Xba*I and *Xho*I of the NB1S3-WT plasmid was respectively replaced for the obtained mutant gene according to operations in the instruction, so as to construct seven expression plasmids NB1S3-K35, NB1S3-T41, NB1S3-K43, NB1S3-Y45, NB1S3-E61, NB1S3-K64 and NB1S3-P65. The mutation was confirmed to be successful by sequencing.

### 4. Construction of rhIL-2 expression strain subjected to site-directed mutation

According to the structure of the plasmid pUltra recorded in the reference (Chatterjee, A. et al., Biochemistry, 52(10), 1828-1837, 2013), tRNA containing a carbonyl-terminal-containing lysine analogue which specifically recognized the structure shown in formula (I) of the present invention and tRNA synthetase encoding genes (a wild-type ancient methanococcus pyrrole lysine synthetase encoding gene and an encoding gene corresponding to tRNA) and a chloramphenicol resistance gene (SEQ ID NO: 46) were obtained by full gene synthesis. Then, a DNA fragment (SEQ ID NO: 47) containing a CloDF13 replication initiation site was amplified from a commercial vector pCDF-duet1 by PCR amplification, and two DNA fragments were further sub-cloned and linked by using a high-fidelity DNA assembly cloning kit to obtain an helper plasmid NBIW (referring to FIG. 2, the plasmid was hereinafter referred to as the helper plasmid). A screening marker of this plasmid was chloramphenicol resistance. The helper plasmid and the expression plasmid (spectinomycin resistance) obtained in the step 3 were jointly transformed into *Escherichia coli* BL21(DE3) respectively, and double-positive strains (the double-positive strains referred to strains obtaining spectinomycin resistance and chloramphenicol resistance at the same time) were screened by spectinomycin resistance and chloramphenicol resistance plates: rhIL2-K35-BL21, rhIL2-T41-BL21, rhIL2-K43-BL21, rhIL2- Y 45-BL21, rhIL2-E61-BL21, rhIL2-K64-BL21, rhIL2-P65-BL21.

The DNA fragment (SEQ ID NO: 46) containing the wild-type ancient *Methanococcus* pyrrole lysine synthetase encoding gene, the encoding gene corresponding to tRNA and the chloramphenicol resistance gene was as follows:

CCTTATGCGACTCCTGCATTAGGGAGCTGTTGACAATTAATCATCGGCTCGTAT
AATGTGTGGAATTGTGAGCGGATAACAATTTCACAAGGAGGTCATATGGATAAAA
AGCCTCTGAACACTCTGATTTCTGCGACCGGTCTGTGGATGTCCCGCACCGGCACCA
TCCACAAAATCAAACACCATGAAGTTAGCCGTTCCAAAATCTACATTGAAATGGCTT
GCGGCGATCACCTGGTTGTCAACAACTCCCGTTCTTCTCGTACCGCTCGCGCACTGCG
CCACCACAAATATCGCAAAACCTGCAAACGTTGCCGTGTTAGCGATGAAGATCTGAA
CAAATTCCTGACCAAAGCTAACGAGGATCAGACCTCCGTAAAAGTGAAGGTAGTAA
GCGCTCCGACCCGTACTAAAAAGGCTATGCCAAAAAGCGTGGCCCGTGCCCCGAAA
CCTCTGGAAAACACCGAGGCGGCTCAGGCTCAACCATCCGGTTCTAAATTTTCTCCG
GCGATCCCAGTGTCCACCCAAGAATCTGTTTCCGTACCAGCAAGCGTGTCTACCAGC
ATTAGCAGCATTTCTACCGGTGCTACCGCTTCTGCGCTGGTAAAAGGTAACACTAAC
CCGATTACTAGCATGTCTGCACCGGTACAGGCAAGCGCCCCAGCTCTGACTAAATCC
CAGACGGACCGTCTGGAGGTGCTGCTGAACCCAAAGGATGAAATCTCTCTGAACAG
CGGCAAGCCTTTCCGTGAGCTGGAAAGCGAGCTGCTGTCTCGTCGTAAAAAGGATCT
GCAACAGATCTACGCTGAGGAACGCGAGAACTATCTGGGTAAGCTGGAGCGCGAAA
TTACTCGCTTCTTCGTGGATCGCGGTTTCCTGGAGATCAAATCTCCGATTCTGATTCC

GCTGGAATACATTGAACGTATGGGCATCGATAATGATACCGAACTGTCTAAACAGAT
CTTCCGTGTGGATAAAAACTTCTGTCTGCGTCCGATGCTGGCCCCGAACCTGTACAA
CTATCTGCGTAAACTGGACCGTGCCCTGCCGGACCCGATCAAAATTTCGAGATCGG
TCCTTGCTACCGTAAAGAGTCCGACGGTAAAGAGCACCTGGAAGAATTCACCATGCT
GAACTTTTGCCAGATGGGTAGCGGTTGCACGCGTGAAAACCTGGAATCCATTATCAC
CGACTTCCTGAATCACCTGGGTATCGATTTCAAAATTGTTGGTGACAGCTGTATGGTG
TACGGCGATACGCTGGATGTTATGCACGGCGATCTGGAGCTGTCTTCCGCAGTAGTG
GGCCCAATCCCGCTGGATCGTGAGTGGGGTATCGACAAACCTTGGATCGGTGCGGGT
TTTGGTCTGGAGCGTCTGCTGAAAGTAAAACACGACTTCAAGAACATCAAACGTGCT
GCACGTTCCGAGTCCTATTACAATGGTATTTCTACTAACCTGTAACTAGTGTCTCCAG
CTTGGCTGTTTTGGCGGATGAGAGAAGATTTTCAGCCTGATACAGATTAAATCAGAA
CGCAGAAGCGGTCTGATAAAACAGAATTTGCCTGGCGGCAGTAGCGCGGTGGTCCC
ACCTGACCCCATGCCGAACTCAGAAGTGAGCACTGCAGCGGCTAACTAAGCGGCCT
GCTGACTTTCTCGCCGATCAAAAGGCATTTTGCTATTAAGGGATTGACGAGGGCGTA
TCTGCGCAGTAAGATGCGCCCCGCATTGGAAACCTGATCATGTAGATCGAATGGACT
CTAAATCCGTTCAGCCGGGTTAGATTCCCGGGGTTTCCGCCAAATTCGAAAAGCCTG
CTCAACGAGCAGGCTTTTTTGCATCTCGAGCAGCTCAGGGTCGAATTTGCTTTCGAAT
TTCTGCCATTCATCCGCTTATTATCACTTATTCAGGCGTAGCAACCAGGCGTTTAAGG
GCACCAATAACTGCCTTAAAAAAATTACGCCCCGCCCTGCCACTCATCGCAGTACTG
TTGTAATTCATTAAGCATTCTGCCGACATGGAAGCCATCACAAACGGCATGATGAAC
CTGAATCGCCAGCGGCATCAGCACCTTGTCGCCTTGCGTATAATATTTGCCCATGGTG
AAAACGGGGGCGAAGAAGTTGTCCATATTGGCCACGTTTAAATCAAAACTGGTGAA
ACTCACCCAGGGATTGGCTGAGACGAAAAACATATTCTCAATAAACCCTTTAGGGAA
ATAGGCCAGGTTTTCACCGTAACACGCCACATCTTGCGAATATATGTGTAGAAACTG
CCGGAAATCGTCGTGGTATTCACTCCAGAGCGATGAAAACGTTTCAGTTTGCTCATG
GAAAACGGTGTAACAAGGGTGAACACTATCCCATATCACCAGCTCACCGTCTTTCAT
TGCCATACGGAATTCCGGATGAGCATTCATCAGGCGGGCAAGAATGTGAATAAAGG
CCGGATAAAACTTGTGCTTATTTTTCTTTACGGTCTTTAAAAAGGCCGTAATATCCAG
CTGAACGGTCTGGTTATAGGTACATTGAGCAACTGACTGAAATGCCTCAAAATGTTC
TTTACGATGCCATTGGGATATATCAACGGTGGTATATCCAGTGATTTTTTTCTCCATT
TTAGCTTCCTTAGCTCCTGAAAATCTCGATAACTCAAAAAATACGCCCGGTAGTGAT
CTTATTTCATTATGGTGAAAGTTGGAACCTCTTACGTGCCGATCAACGTCTCATTTTC
GCCAAAAGTTGGCCCAGGGCTTCCCGGTATCAACAGGGACACCAGGATTTATTTATT
CTGCGAAGTGATCTTCCGTCACAGGTATTTATTCGGCGCAAAGTGCGTCGGGTGATG
CTGCCAACTTACTGATTTAGTGTATGATGGTGTTTTTGAGGTGCTCCAGTGGCTTCTG

TTTCTATCAGCTGTCCCTCCTGTTCAGCTACTGACGGGGTGGTGCGTAACGGCAAAA
GCACCGCCGGACATCAGCGCGCTGCGGACAC

The DNA fragment (SEQ ID NO: 47) of the CloDF13 replication initiation site was as follows:

CGGACATCAGCGCGCTGCGGACACATACAAAGTTACCCACAGATTCCGTGGA
TAAGCAGGGGACTAACATGTGAGGCAAAACAGCAGGGCCGCGCCGGTGGCGTTTT
CCATAGGCTCCGCCCTCCTGCCAGAGTTCACATAAACAGACGCTTTTCCGGTGCATCT
GTGGGAGCCGTGAGGCTCAACCATGAATCTGACAGTACGGGCGAAACCCGACAGGA
CTTAAAGATCCCCACCGTTTCCGGCGGGTCGCTCCCTCTTGCGCTCTCCTGTTCCGAC
CCTGCCGTTTACCGGATACCTGTTCCGCCTTTCTCCCTTACGGGAAGTGTGGCGCTTT
CTCATAGCTCACACACTGGTATCTCGGCTCGGTGTAGGTCGTTCGCTCCAAGCTGGG
CTGTAAGCAAGAACTCCCCGTTCAGCCCGACTGCTGCGCCTTATCCGGTAACTGTTC
ACTTGAGTCCAACCCGGAAAAGCACGGTAAAACGCCACTGGCAGCAGCCATTGGTA
ACTGGGAGTTCGCAGAGGATTTGTTTAGCTAAACACGCGGTTGCTCTTGAAGTGTGC
GCCAAAGTCCGGCTACACTGGAAGGACAGATTTGGTTGCTGTGCTCTGCGAAAGCCA
GTTACCACGGTTAAGCAGTTCCCCAACTGACTTAACCTTCGATCAAACCACCTCCCC
AGGTGGTTTTTTCGTTTACAGGGCAAAAGATTACGCGCAGAAAAAAAGGATCTCAAG
AAGATCCTTTGATCTTTTCTACTGAACCGCTCTAGATTTCAGTGCAATTTATCTCTTCA
AATGTAGCACCTGAAGTCAGCCCCATACGATATAAGTTGTAATTCTCATGTTAGTCA
TGCCCCGCGCCCACCGGAAGGAGCTGACTGGGTTGAAGGCTCTCAAGGGCATCGGTC
GAGATCCCGGTGCCTAATGAGTGAGCTAACTTACATTAATTGCGTTGCGCTCACTGC
CCGCTTTCCAGTCGGGAAACCTGTCGTGCCAGCTGCATTAATGAATCGGCCAACGCG
CGGGGAGAGGCGGTTTGCGTATTGGGCGCCAGGGTGGTTTTTCTTTTCACCAGTGAG
ACGGGCAACAGCTGATTGCCCTTCACCGCCTGGCCCTGAGAGAGTTGCAGCAAGCGG
TCCACGCTGGTTTGCCCCAGCAGGCGAAAATCCTGTTTGATGGTGGTTAACGGCGGG
ATATAACATGAGCTGTCTTCGGTATCGTCGTATCCCACTACCGAGATGTCCGCACCA
ACGCGCAGCCCGGACTCGGTAATGGCGCGCATTGCGCCCAGCGCCATCTGATCGTTG
GCAACCAGCATCGCAGTGGGAACGATGCCCTCATTCAGCATTTGCATGGTTTGTTGA
AAACCGGACATGGCACTCCAGTCGCCTTCCCGTTCCGCTATCGGCTGAATTTGATTGC
GAGTGAGATATTTATGCCAGCCAGCCAGACGCAGACGCGCCGAGACAGAACTTAAT
GGGCCCGCTAACAGCGCGATTTGCTGGTGACCCAATGCGACCAGATGCTCCACGCCC
AGTCGCGTACCGTCTTCATGGGAGAAAATAATACTGTTGATGGGTGTCTGGTCAGAG
ACATCAAGAAATAACGCCGGAACATTAGTGCAGGCAGCTTCCACAGCAATGGCATC
CTGGTCATCCAGCGGATAGTTAATGATCAGCCCACTGACGCGTTGCGCGAGAAGATT
GTGCACCGCCGCTTTACAGGCTTCGACGCCGCTTCGTTCTACCATCGACACCACCAC
GCTGGCACCCAGTTGATCGGCGCGAGATTTAATCGCCGCGACAATTTGCGACGGCGC

GTGCAGGGCCAGACTGGAGGTGGCAACGCCAATCAGCAACGACTGTTTGCCCGCCA
GTTGTTGTGCCACGCGGTTGGGAATGTAATTCAGCTCCGCCATCGCCGCTTCCACTTT
TTCCCGCGTTTTCGCAGAAACGTGGCTGGCCTGGTTCACCACGCGGGAAACGGTCTG
ATAAGAGACACCGGCATACTCTGCGACATCGTATAACGTTACTGGTTTCACATTCAC
CACCCTGAATTGACTCTCTTCCGGGCGCTATCATGCCATACCGCGAAAGGTTTTGCGC
CATTCGATGGTGTCCGGGATCTCGACGCTCTCCCTTATGCGACTC

### Example 2 Expression and purification of rhIL-2 inserted with unnatural amino acid subjected to site-directed mutation

### 1. Expression of mutant rhIL-2 incorporating with unnatural amino acid

Seven expression strains rhIL2-K35-BL21, rhIL2-T41-BL21, rhIL2-K43-BL21, rhIL2-Y45-BL21, rhIL2-E61-BL21, rhIL2-K64-BL21 and rhIL2-P65-BL21 obtained in Example 1 were respectively inoculated into a LB medium (5 g/L yeast extract, 10 g/L tryptone and 10 g/L NaCl, containing 100 mg/L spectinomycin and 37.5 mg/L chloramphenicol), cultured at 37°C for 5 hours to 8 hours, and then subjected to secondary expanded inoculation (in a medium with the same composition as the previous medium) until OD₆₀₀ of the bacterial solution was 2.0±0.2 to obtain a secondary seed solution.

The above secondary seed solution was inoculated into a fermentation medium for fermentation culture in a 5 L fermentor. The culture volume was 2 L, the medium was 2×YT medium (16 g/L yeast extract, 10 g/L tryptone and 5 g/L NaCl), and the inoculation amount was 5% (v/v); the culture temperature was 37°C; the pH value was controlled to be 6.90±0.05, and ammonia water or H₃PO₄ was automatically added when necessary; DO was controlled to be 30%, and DO was related to the rotating speed. When OD₆₀₀ of the bacterial solution reached 20.0±2.0, IPTG and the unnatural amino acid NBOK obtained in Preparation Example 1 were added, with final concentrations of 1 mM. Meanwhile, 50% glycerol started to be fed at a feeding speed of 0.6±0.1 mL/min. After inducing expression for 5 hours to 6 hours, bacterial cells were collected. SDS-PAGE electrophoregrams of various strains were shown in FIG. 3.

### 2. Isolation and extraction of mutant rhIL-2

The collected bacterial cells above were respectively suspended with a buffer solution (25 mM Tris, 6 mM EDTA and 1 mM DTT, at pH 8.0), added with 1% DNase (1 mg/mL) and 0.5% PMSF, mixed evenly, and homogenized for 3 times with an ultrahigh-pressure homogenizer under a pressure of 50 MPa to 80 MPa. The homogenate was centrifuged at 10,000 rpm for 20 minutes, and the lower crude inclusion bodies were collected.

The obtained crude inclusion bodies were washed twice with a washing buffer (20 mM Tris-HCl, 100 mM NaCl and 2% TritonX-100, at pH 8.0), and then washed once with ultrapure water to obtain purified inclusion bodies.

The purified inclusion bodies were dissolved with a denaturation buffer (20 mM Tris-HCl, 100 mM NaCl, 6 M guanidine hydrochloride and 1 mM DTT, at pH 8.0), and centrifuged at 10,000 rpm 30 minutes later, and the supernatant was collected, which was a denatured protein solution. A renaturation buffer (20 mM Tris-HCl and 100 mM NaCl, at pH 8.0) in a volume 4 times of the volume of the solution was added into the collected denatured protein solution, fully stirred and then allowed to stand for 12 hours, and centrifuged at 10,000 rpm to collect the supernatant, which was a renaturated protein solution.

The renaturated protein solution was concentrated to 1/4 of the original volume with an ultrafiltration cassette (Millipore, Biomax-5) with a molecular weight cut-off of 5 kDa, medium was substituted with a substitution buffer (20 mM Tris-HCl, at pH 8.0) until conductivity reached about 2 ms/cm, the solution was further concentrated until a protein concentration was about 0.5 mg/mL to 1 mg/mL, and centrifuged at 10,000 rpm, and then a supernatant was collected, so that crude proteins of mutant rhIL-2 were obtained, including rhIL2-K35, rhIL2-T41, rhIL2-K43, rhEL2-Y45, rhIL2-E61, rhIL2-K64 and rhIL2-P65, and could be directly used for subsequent PEG coupling.

### Example 3 Site-directed coupling between PEG and rhIL-2 inserted with unnatural amino acid NBOK subjected to site-directed mutation

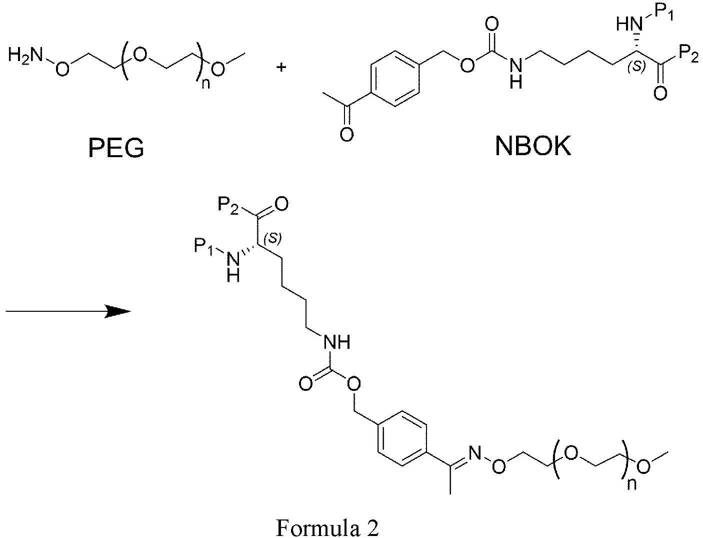

The site-directed coupling between the PEG and the rhIL-2 inserted with the unnatural amino acid subjected to site-directed mutation was shown in the synthetic route of Formula 2 (wherein the direction from of P₁ to P₂ was the direction from the N-terminal to the C-terminal of the amino acid sequence).

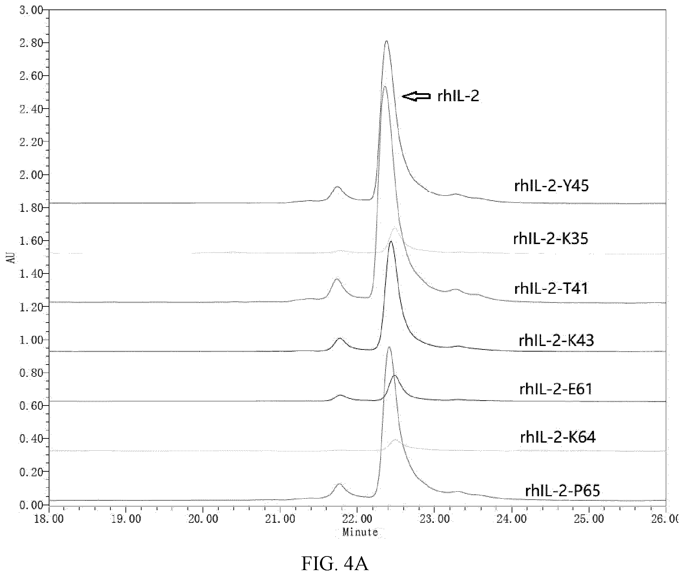

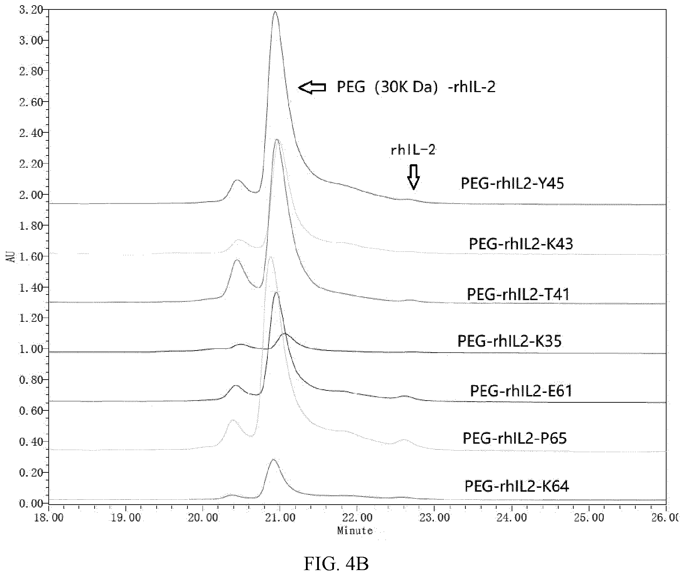

Taking the oximation reaction of 30 KD aminoxy PEG (i.e. hydroxyamino PEG) coupled with the rhIL-2 as an example, the coupling reaction was operated as follows: before the coupling reaction, the obtained target protein above was adjusted with 2 M acetic acid solution until the pH value was 4.0, adjusted with 20 mM sodium acetate buffer (at pH 4.0) until the protein concentration was about 1 mg/ml, added with a 30 KD aminoxy PEG solid (purchased from Beijing Jenkem Technology Co., Ltd.) according to 1:15 (a molar ratio of the protein to the aminoxy PEG), and fully shaken for dissolution to obtain a clear and transparent solution. Subsequently, the reaction solution was blocked, and shaken in a constant temperature shaker (25°C, 100 rpm) for reaction. The coupling progress was analyzed by RP-HPLC 48 hours later, as shown in FIG. 4A and FIG. 4B. Results showed that there was only a weak peak of rhIL-2 in FIG. 4B, which indicated that the seven target proteins were all coupled with the PEG, and coupling rates (100% - residual rhIL-2 concentration after coupling / rhIL-2 concentration when the coupling reaction time was zero ×100%) were all greater than 95%, thus further indicating that the unnatural amino acid NBOK above was inserted into the target protein.

The mutant rhIL-2 proteins coupled with the PEG were respectively named as: 30KD PEG-rhII,2-K35, 30KD PEG-rhIL2-T41, 30KD PEG-rhII,2-K43, 30KD PEG-rhII,2-Y45, 30KD PEG-rhII,2-E61, 30KD PEG-rhII,2-K64 and 30KD PEG-rhIL2-P65.

RP-HPLC analysis conditions were as follows:
mobile phase A (0.1% TFA-H₂O); and
mobile phase B (0.1% TFA-ACN).

| | | | |
|---|---|---|---|
| Chromatographic column | ZORBAX 300SB-C8, 4.6×150 mm, 5 µm | | |
| Detection wavelength | 214 nm | | |
| Temperature of column thermerstat | 30°C | | |
| Temperature of sample injector | 15°C | | |
| Flow velocity | 1.0 mL/min | | |
| Gradient elution | Time (min) | A (%) | B (%) |
| | 0 | 90 | 10 |
| | 10 | 65 | 35 |
| | 30 | 20 | 80 |
| | 31 | 90 | 10 |
| | 45 | 90 | 10 |

### Example 4 Purification of 30KD PEG-rhIL2 subjected to site-directed modification

Chromatographic medium: Capto MMC; balance buffer: 20 mM sodium citrate buffer (pH=3.0), and elution buffer: 20 mM sodium citrate buffer - 1 M NaCl (pH=7.8).

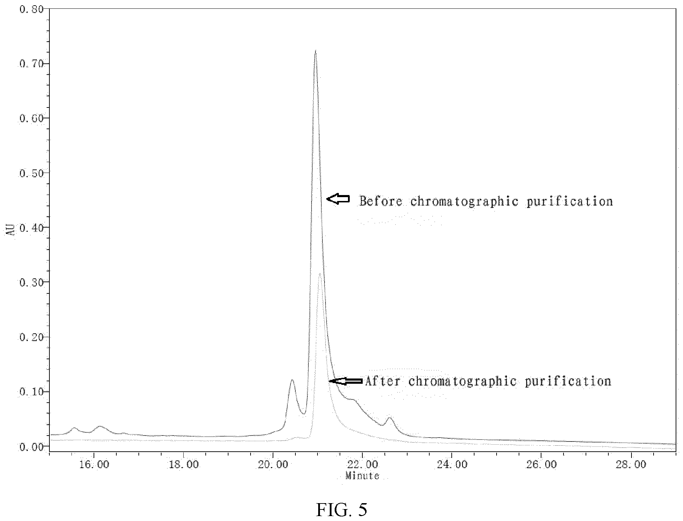

The purification process specifically included: adjusting 30KD PEG-rhII,2-K35, 30KD PEG-rhIL2-T41, 30KD PEG-rhII,2-K43, 30KD PEG-rhII,2-Y45, 30KD PEG-rhII,2-E61, 30KD PEG-rhII,2-K64 and 30KD PEG-rhIL2-P65 coupling reaction solutions obtained in Example 3 with balance buffer respectively until the pH value was 3.0±0.2, with the conductivity less than or equal to 5 ms/cm, loading samples to Capto MMC, carrying out linear elution with an elution buffer (0-100% eluent, 20 CV), and collecting target protein components, so as to obtain target protein samples with a purity of about 95%. Taking 30KD PEG-rhIL2-Y45 as an example, typical RP-HPLC profile of conjugate 30KD PEG-rhIL2-Y45 after purification and rhIL2-Y45 before coupling were shown in FIG. 5. Other protein samples had similar purification results.

### Example 5 In-vitro activity evaluation on 30KD PEG-rhIL2 subjected to site-directed modification (STAT5 phosphorylation assay)

In this method, two cell lines were adopted, wherein mouse CTLL-2 cells were a cell line containing IL-2Rαβγ, and human YT cells were a cell line containing IL-2Rβγ, and rhIL-2 activated the JAK-STAT signal pathway by binding with IL-2R on the cell surface. Different modification sites of various samples had different relative activities to the two kinds of cells. The lower the percentage change of EC50 ratio of YT cells / CTLL-2 cells was, the better the effect of promoting the immune function of the samples was, and on the contrary, the better the effect of inhibiting the immune function was.

The specific process was as follows: the mouse CTLL-2 cells (purchased from American Type Culture Collection) and the human YT cells were cultured in each specific media (medium for CTLL-2 cell: RPMI 1640+10% FBS+400 IU/mL rhIL-2, 2 mM L-glutamine and 1 mM sodium pyruvate; medium for YT cell: RPMI 1640+10% FBS+1 mm Non-Essential Amino Acids Solution (purchased from Gibco, product catalog number 11140050)) to a sufficient amount at condition of 37°C and 5% carbon dioxide, and starved for 4 hours before detection, and then the cell density was adjusted to be 1 × 10⁶ cells/mL for later use. 30KD PEG-rhIL2-K35, 30KD PEG-rhIL2-T41, 30KD PEG-rhIL2-Y45, 30KD PEG-rhIL2-E61 and 30KD PEG-rhIL2-P65, samples of rhIL2-K35, rhIL2-T41, rhIL2-Y45, rhIL2-E61 and rhIL2-P65 before coupling, and a reference rhIL-2 (purchased from Beijing Solarbio Science & Technology Co., Ltd., catalog number: P00020) were gradiently diluted respectively, and each sample had a total of six concentrations (in the CTLL-2 cell assay, the reference had a concentration range of 0.004 ng/mL to 4 ng/mL, and was subjected to 4-fold gradient dilution; in the YT cell assay, the reference had a concentration range of 2.1 ng/mL to 510 ng/mL, and was subjected to 3-fold gradient dilution; and other samples had a concentration range obtained through pre-experimental screening, which corresponded to corresponding EC50 values in Table 2). The cells were stimulated at 37°C for 10 minutes and then lysed, a western blot experiment was carried out, the cells were hybridized with a pSTAT5 antibody (purchased from CST, product catalog number 9359L) and β-actin (purchased from CST, product catalog number 8457S), protein amounts of pSTAT5 and β-actin in the cell lysate were detected, and EC50 was calculated according to gray scale results of pSTAT5/β-actin and sample concentrations. Results were shown in Table 2. The results showed that rhIL2 inserted with unnatural amino acids NBOK at the K35 site, the T41 site, the Y45 site, the E61 site and the P65 site and coupled with 30 KD PEG all met original design requirements (compared with the reference, the percentage change of EC50 ratio was lower than that of the reference).

**Table 2 Results of STATS phosphorylation assay**

| STAT5 phosphorylation assay (CTLL-2 cells) | | | STAT5 phosphorylation assay (YT cells) | | | Percentage change of EC50 ratio of YT/CTLL-2 cells of 30KD PEG-rhIL2 sample* |
|---|---|---|---|---|---|---|
| Mutation site | EC50 (ng/mL) | | Mutation site | EC50 (ng/mL) | | |
| | rhIL-2 | 30KD PEG-rhIL2 | | rhIL-2 | 30KD PEG-rhIL2 | |
| Reference | 0.18 | / | Reference | 24.5 | / | 100% |
| K35 | 0.17 | 1.54 | K35 | 78.1 | 169.5 | 81% |
| T41 | 0.35 | 3.08 | T41 | 26.4 | 189.0 | 45% |
| Y45 | 1.06 | 36.2 | Y45 | 49.4 | 198.5 | 4% |
| E61 | 0.33 | 3.67 | E61 | 49.9 | 389.1 | 78% |
| P65 | 0.09 | 4.17 | P65 | 57.0 | 409.5 | 72% |

| | | | | | | |
|---|---|---|---|---|---|---|
| *Calculation formula: [EC50 of YT cells of 30KD PEG-rhIL2 sample/EC50 of CTLL-2 cells of 30KD PEG-rhIL2 sample] / [EC50 of YT cells of reference / EC50 of CTLL-2 cells of reference] ×100% | | | | | | |

### Example 6 In-vivo pharmacokinetic study in mouse

Female C57 mice (SPF grade, purchased from Zhejiang Vital River Laboratory Animal Technology Co., Ltd.) were used in the experiment, Quanqi rhIL-2 (purchased from Shandong Quangang Pharmaceutical Co., Ltd.) was used as a positive control drug, and the metabolism situation of 30KD PEG-rhIL2-Y45 in the mice was investigated. The 30KD PEG-rhIL2-Y45 and the Quanqi rhIL-2 were respectively administrated by single intravenous injection according to 1 mg/kg, and blood sampling was carried out according to the following blood sampling points, with 0.5 mL (n=5) for each point: before administration, and 0.0833 hour, 0.5 hour, 1 hour, 4 hours, 8 hours, 16 hours and 24 hours after administration. In addition, additional 5 blood sampling points were set for the 30KD PEG-rhIL2-Y45: 48 hours, 72 hours, 96 hours, 120 hours and 144 hours. The blood sample was placed at room temperature for 15 minutes and centrifuged at 6,800 g/min for 6 minutes to obtain serum. The plasma concentration in the serum was analyzed by the following method:
(1) Coating: 50 µL of 1 µg/mL working solution of Anti-IL-2 antibody (purchased from abcam, product catalog number: ab9618) was added into each well in a high-adsorption 96-well plate, and incubated at 2°C to 8°C overnight. (2) Washing: the liquid in the well was discarded, and the well was washed with 1×PBST (0.05% Tween-20) for 3 times, according to 300 µL/well. (3) Blocking: a casein blocking solution (purchased from Thermo, product catalog number 37528) was added according to 200 µL/well, and allowed to stand at room temperature for 90 minutes. (4) Washing: the liquid in the well was discarded, and the well was washed with 1×PBST for 3 times, according to 300 µL/well. (5) Sample loading: the Quanqi rhIL-2, the 30KD PEG-rhIL2-Y45 and the serum sample to be tested were gradiently diluted with mouse serum, transferred to a micro-well plate according to 50 µL/well, and allowed to stand at room temperature for 120 minutes. (6) Washing: the liquid in the well was discarded, and the well was washed with 1×PBST for 3 times, according to 300 µL/well. (7) Primary antibody: 50 µL of 0.25 µg/mL working solution of IL-2 Monoclonal Antibody (BG5) and Biotin (purchased from Invitrogen, product catalog number M600B) was added into each well, and allowed to stand at room temperature for 60 minutes. (8) Washing: the liquid in the well was discarded, and the well was washed with 1×PBST for 3 times, according to 300 µL/well. (9) Secondary antibody: Pierce^{TM} High Sensitivity Streptavidin-HRP (purchased from Thermo, product catalog number 21130) was diluted for 4,000 times with a casein blocking solution, and 50 µL was added into each well, and allowed to stand at room temperature for 60 minutes. (10) Washing: the liquid in the well was discarded, and the well was washed with 1×PBST for 4 times, according to 300 µL/well. (11) Substrate: 50 µL of 1-Step^{TM} Turbo TMB-ELISA Substrate Solution (purchased from Thermo, product catalog number 34022) was added into each well. (12) Stopping and reading: 2 M sulfuric acid stop solution was added 25 minutes later, and light absorption values at 450 nm and 650 nm were read on a microplate reader (purchased from Perkin Elmer, model EnSight). (13) Analysis: four-parameter fitting was carried out by Dazdaq Ltd. WorkOut 1.5 analysis software, and concentrations and units corresponding to the Quanqi rhIL-2 and the 30KD PEG-rhIL2-Y45 were input to fit a curve for each of the two, so as to respectively calculate the plasma concentration in the serum to be tested. According to the non-compartment model (statistical moment parameter) of DAS software, average half-life t_{1/2} of Quanqi rhIL-2 and 30KD PEG-rhIL2-Y45 were calculated to be 0.83 hour and 19.83 hours respectively. The pharmacokinetic parameters of Quanqi rhIL-2 and 30KD PEG-rhIL2-Y45 were shown in Table 3.

**Table 3 Pharmacokinetic parameters (n=5) of Quanqi rhIL-2 and 30KD PEG-rhIL2-Y45**

| Pharmacokinetic parameter (n=5) | Quanqi rhIL-2 | | 30KD PEG-rhIL2-Y45 | |
|---|---|---|---|---|
| Parameter | Average value | Standard deviation | Average value | Standard deviation |
| AUC₀₋ₜ (ng/mL×h) | 2815 | 439 | 156482 | 22038 |
| MRT₀₋ₜ (h) | 0.43 | 0.058 | 9.67 | 0.371 |
| t_{1/2} (h) | 0.83 | 0.354 | 19.83 | 9.192 |
| Tₘₐₓ (h) | 0.083 | 0.000 | 0.500 | 0.000 |
| CL (L/h/kg) | 0.406 | 0.065 | 0.007 | 0.001 |
| Vz (L/kg) | 0.506 | 0.277 | 0.194 | 0.121 |
| Cₘₐₓ (ng/mL) | 6627 | 1398 | 25994 | 579 |

### Example 7 In-vivo pharmacodynamical study in mouse - anti-tumor activity

Female Balb/c mice (SPF grade, Zhejiang Vital River Laboratory Animal Technology Co., Ltd.) were used in the experiment. A cell suspension of 2 × 10⁵/0.1mL/mouse CT26.WT (purchased from ATCC, product catalog number CRL-2638) and a cell suspension of 4 ×10⁵/0.1mL/mouse H22 (purchased from CCTCC, product catalog number GDC0091) were subcutaneously inoculated into right backs of the mice. When the tumor volume reached about 50 mm³, the mice were randomly grouped, with seven mice in each group, and respectively administrated with vehicle (1×PBS), 0.7 mg/kg 30KD PEG-rhIL2-T41, 5.0 mg/kg 30KD PEG-rhIL2-T41, 0.7 mg/kg 30KD PEG-rhII,2-Y45 and 5.0 mg/kg 30KD PEG-rhII,2-Y45 (with administration volumes of 10 mL/kg). During the experiment, weights and tumor volumes of the animals were measured 3 times every week, and administration modes and experimental results were shown in Table 4. Calculation formula of relative inhibition rate TGI_{TW} (%): (T_{WC}-T_{WT})/T_{WC}×100%, wherein Twc was the average tumor weight of the vehicle control group, and T_{WT} was the average tumor weight of the treatment group.

Results showed that, compared with the vehicle group, high-dosage and low-dosage groups of the two test substances had significant inhibitory effects on homograft tumors of mouse colon cancer CT26.WT and mouse liver cancer H22.

**Table 4 Administration modes of test substances in homograft tumor models of CT26.WT and H22, and influences of test substances on tumor weights of animals**

| | | Administration mode | | | CT26.WT model | | H22 model | |
|---|---|---|---|---|---|---|---|---|
| Group | Test substanc e | Administra tion dosage (mg/kg) | Administra tion route | Administrati on frequency | Average value±standard error of tumor weight (g) | TGI_{TW} (%) | Average value±standard error of tumor weight (g) | TGI_{TW} (%) |
| 1 | Solvent control | N/A | IV | Q5D×3 | 3.17±0.18 | / | 1.64±0.13 | / |
| 2 | High dosage of 30KD PEG-rhI L2-T41 | 5.0 | IV | Q5D×3 | 0.47±0.21 *** | 85 | 0.34±0.12 *** | 79 |
| 3 | Low dosage of 30KD PEG-rhI L2-T41 | 0.7 | IV | Q5D×3 | 0.73±0.07 *** | 77 | 0.66±0.07 *** | 60 |
| 4 | High dosage of 30KD PEG-rhI L2-Y45 | 5.0 | IV | Q5D×3 | 0.37±0.19 *** | 88 | 0.38±0.13 *** | 77 |
| 5 | Low dosage of 30KD PEG-rhI L2-Y45 | 0.7 | IV | Q5D×3 | 1.15±0.24 *** | 64 | 0.72±0.15 *** | 56 |

| | | | | | | | | |
|---|---|---|---|---|---|---|---|---|
| Note: *: p<0.05; **: p<0.01; ***: p<0.001, compared with the vehicle group. | | | | | | | | |

### Example 8 In-vivo pharmacodynamical study in mouse - expression of immune cell population

Female Balb/c mice (SPF grade, Zhejiang Vital River Laboratory Animal Technology Co., Ltd.) were used in the experiment. A cell suspension of 2 × 10⁵/0.1 mL/mouse CT26.WT was subcutaneously inoculated into right backs of the mice. When the tumor volume reached about 100 mm³, the mice were randomly grouped, with 3 mice in each group, and respectively administrated with various test substances (wherein Quanqi^{®} was a commercially available recombinant human IL-2 injection) according to Table 5, with administration volumes of 10 mL/kg. On the 5^{th} day, tumor tissue samples of various groups were collected for flow cytometry detection of changes in cell populations of CD8⁺ T cells and CD4⁺ Treg cells. Results were shown in Table 5.

**Table 5 Administration mode of test substances in homograft tumor model of CT26.WT, and influence of test substances on immune cell population**

| Group | Test substance | Administra tion dosage (mg/kg) | Administrati on route | Administration frequency | Percentage of each cell population in leucocytes (%) | | |
|---|---|---|---|---|---|---|---|
| | | | | | CD8⁺ T cells | CD4⁺ Treg | CD8⁺ T cells/ CD4⁺Treg |
| 1 | Vehicle control | N/A | IV | Single dosage | 4.1 | 1.8 | 2.3 |
| 2 | Quanqi^{®} | 2.0 | IP | QD×5 | 9.7 | 6.9 | 1.4 |
| 3 | 30KD PEG-rhIL2-T 41 | 2.0 | IV | Single dosage | 40.3 | 1.5 | 26.9 |
| 4 | 30KD PEG-rhIL2-Y 45 | 2.0 | IV | Single dosage | 28.5 | 1.4 | 20.4 |

Compared with the Quanqi control, the 30KD PEG-rhIL2-T41 and the 30KD PEG-rhIL2-Y45 had significantly increased proportions of CD8⁺ T cells, significantly decreased proportions of CD4⁺ Treg cells, and significantly increased proportions of CD8⁺ T/ CD4⁺ Treg, showing excellent immune enhancement effects.

### Example 9 Reduction of active group of Lys-azido

With reference to the method in Example 1, an expression strain of rhIL-2 (rhGH-V91 for short) in which the amino acid (valine) codons at the 91^{st} site were mutated into amber codons was constructed, and primers used in the construction process were as follows:
V91-F: 5'-GATTTCCAATATCAACTAGATTGTTCTGGAACTGA-3' (SEQ ID NO: 48)
V91-R: 5'-TCAGTTCCAGAACAATCTAGTTGATATTGGAAATC-3' (SEQ ID NO: 49).

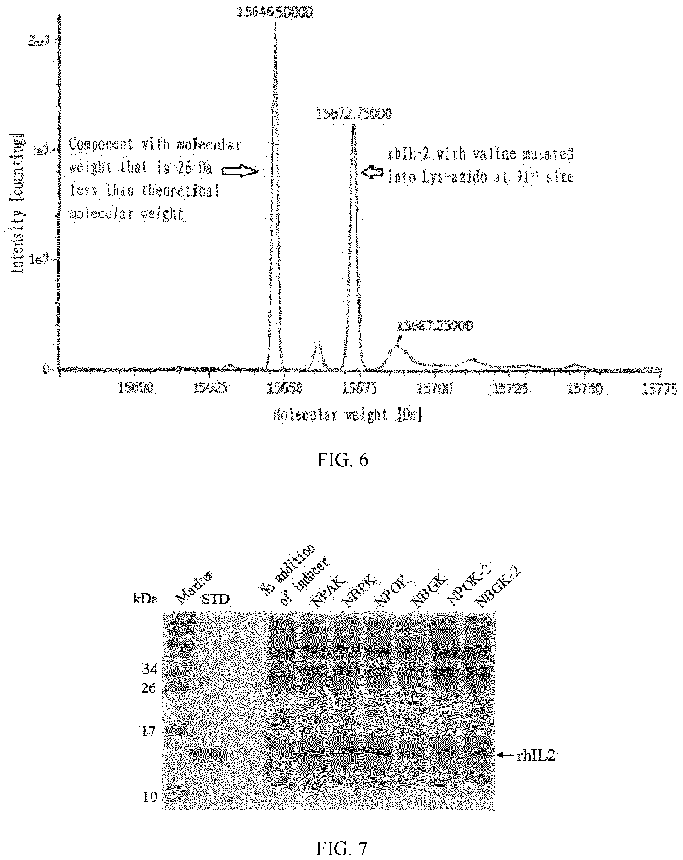

With reference to Example 2, the above rhGH-V91 expression strain was used to express the rhIL-2 subjected to mutation to Lys-azido at the 91^{st} site by adding Lys-azido in the fermentation process, and purification was carried out by the corresponding purification method in Example 2. The complete molecular weight of the rhIL-2 subjected to mutation to Lys-azido at the 91^{st} site was analyzed by liquid chromatography-mass spectrometry (high resolution mass spectrometer: XevoG2-XS Q-Tof, Waters Company; ultra-high performance liquid chromatography: UPLC (Acquity UPLC I-Class, Waters Company), as shown in FIG. 6. Results showed that there was a component which was about 26 Da smaller than the theoretical molecular weight (15672.75 Da) in the sample, and it was inferred that the azido structure (-N₃) at the terminal of Lys-azido was reduced to the product of (-NH₂).

The reduction of Lys-azido could lead to the failure to couple the rhIL-2 subjected to mutation to Lys-azido with BCN-PEG, thus reducing the coupling rate. However, when the rhIL-2 containing the unnatural amino acid of the present invention was coupled with PEG, the coupling rate was significantly increased, thus significantly improving the reaction efficiency.

### Example 10 Expression and purification of rhIL-2 inserted with different unnatural amino acids subjected to site-directed mutation

### 1. Expression of mutant rhIL-2 incorporating unnatural amino acid

The expression strain rhIL2-T41-BL21 obtained in Example 1 was inoculated into a LB medium (5 g/L yeast extract, 10 g/L tryptone and 10 g/L NaCl, containing 100 mg/L spectinomycin), cultured at 37°C for 4 hours to 6 hours, and then subjected to secondary expanded inoculation (in a medium being a LB medium) until OD₆₀₀ of the bacterial solution was 2.0±0.2 to obtain a secondary seed solution.

The above secondary seed solution was inoculated into a fermentation medium for fermentation culture in a 3 L quadruple fermentor. The culture volume was 1 L, the medium was 2×YT medium (16 g/L yeast extract, 10 g/L tryptone and 5 g/L NaCl), and the inoculation amount was 5% (v/v); the culture temperature was 37°C; the pH value was controlled to be 6.90±0.05, and ammonia water or H₃PO₄ was automatically added when necessary; DO was controlled to be 30%, and DO was related to the rotating speed. When OD₆₀₀ of the bacterial solution reached 20.0±2.0, IPTG and the unnatural amino acids obtained in Preparation Example 2 (NPAK), Preparation Example 3 (NBPK), Preparation Example 4 (NPOK), Preparation Example 5 (NBGK), Preparation Example 6 (NPOK-2) and Preparation Example 7 (NBGK-2) were added respectively, with final concentrations of 1 mM. Meanwhile, 50% glycerol started to be fed at a feeding speed of 0.5±0.1 mL/min. After inducing expression for 5 hours to 6 hours, bacterial cells were collected. SDS-PAGE electrophoretograms of recombinant IL-2 inserted with different unnatural amino acids were shown in FIG. 7.

### 2. Isolation and extraction of mutant rhIL-2

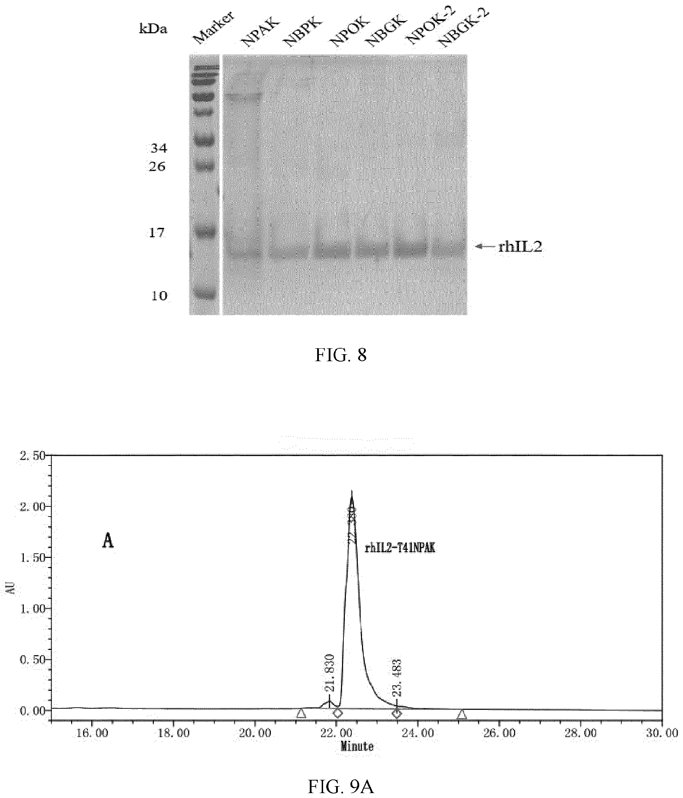

The collected bacterial cells above were respectively isolated and extracted according to the isolation and extraction methods described in Example 2 to obtain crude proteins of mutant rhIL-2 with different unnatural amino acids (referring to FIG. 8), which were respectively named rhIL2-T41NPAK, rhIL2-T41NBPK, rhIL2-T41NPOK, rhIL2-T41NBGK, rhIL2-T41NPOK-2 and rhIL2-T41NBGK-2, and could be directly used for subsequent PEG coupling.

### Example 11 Site-directed coupling between PEG and rhIL-2 inserted with unnatural amino acid NPAK subjected to site-directed mutation

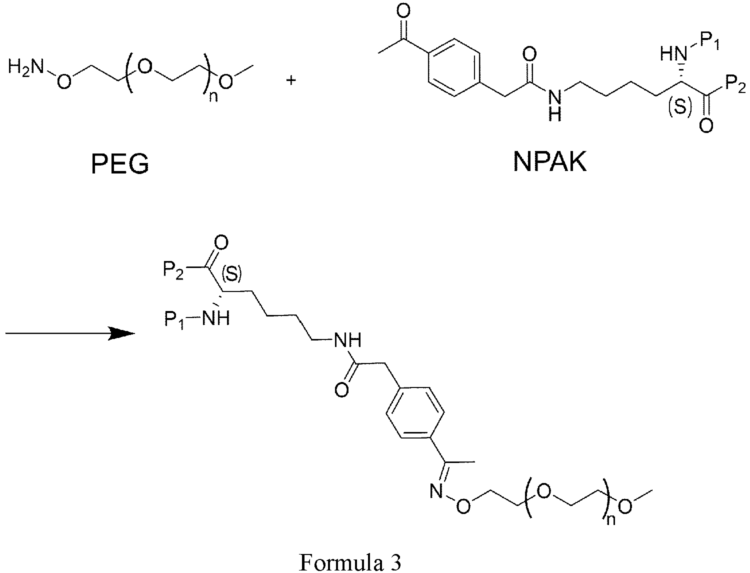

The site-directed coupling between the PEG and the rhIL-2 inserted with the NPAK subjected to site-directed mutation (rhIL2-T41NPAK) was shown in the synthetic route of Formula 3 (wherein the direction from of P₁ to P₂ was the direction from the N-terminal to the C-terminal of the amino acid sequence).

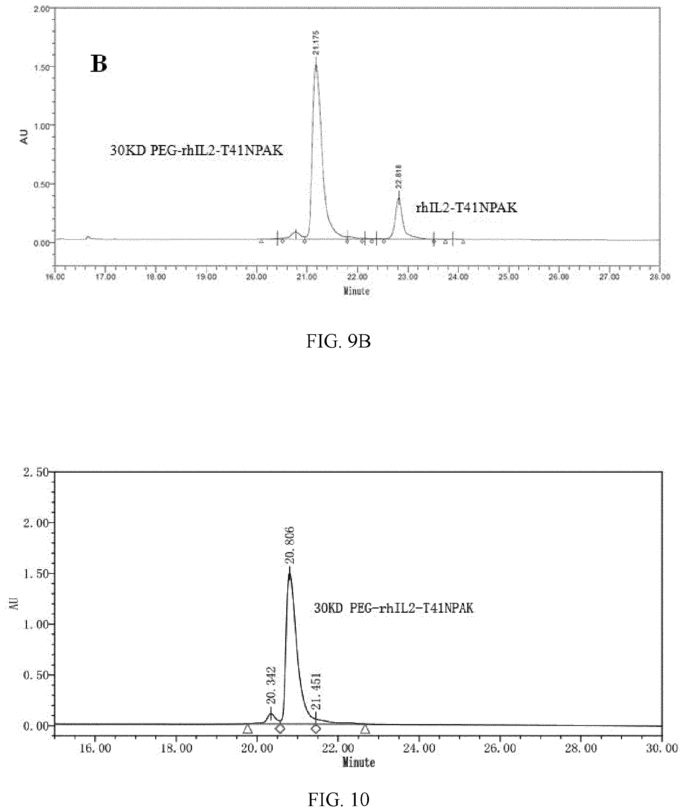

Taking the oximation reaction of 30 KD aminoxy PEG (i.e. hydroxylamine PEG) coupled with the rhIL-2 as an example, the operation of the coupling reaction was the same as that of Example 3. The coupling situation was analyzed by RP-HPLC after the coupling reaction, RP-HPLC analysis conditions were the same as those of Example 3, and analysis results were shown in FIG. 9A and FIG. 9B. The results showed that the peak of rhIL-2 was greatly decreased compared with that before the reaction in FIG. 9B, and the peak of product (21.175 min) was greatly increased correspondingly, which indicated that the target protein was coupled with the PEG, and the coupling rate (100% - residual rhIL-2 concentration after coupling / rhIL-2 concentration when the coupling reaction time was zero × 100%) was nearly 80%.

The protein of mutant rhIL-2 coupled with the PEG was named as 30KD PEG-rhIL2- T 41 NP AK.

### Example 12 Purification of 30KD PEG-rhIL2-T41NPAK subjected to site-directed modification

The 30KD PEG-rhIL2-T41NPAK obtained in Example 11 was purified according to the purification method described in Example 4 to obtain a target protein sample with a purity of about 95%. The typical RP-HPLC profile of the purified conjugate was shown in FIG. 10.

### Example 13 In-vitro activity evaluation on 30KD PEG-rhIL2-T41NPAK subjected to site-directed modification (STAT5 phosphorylation assay)

In-vitro activity evaluation was carried out on the 30KD PEG-rhIL2-T41NPAK obtained in Example 12 according to the evaluation method described in Example 5, and the only difference from the evaluation method in Example 5 was that: the concentration range of the reference was 6.3 ng/mL to 1530 ng/mL in the YT cell assay.

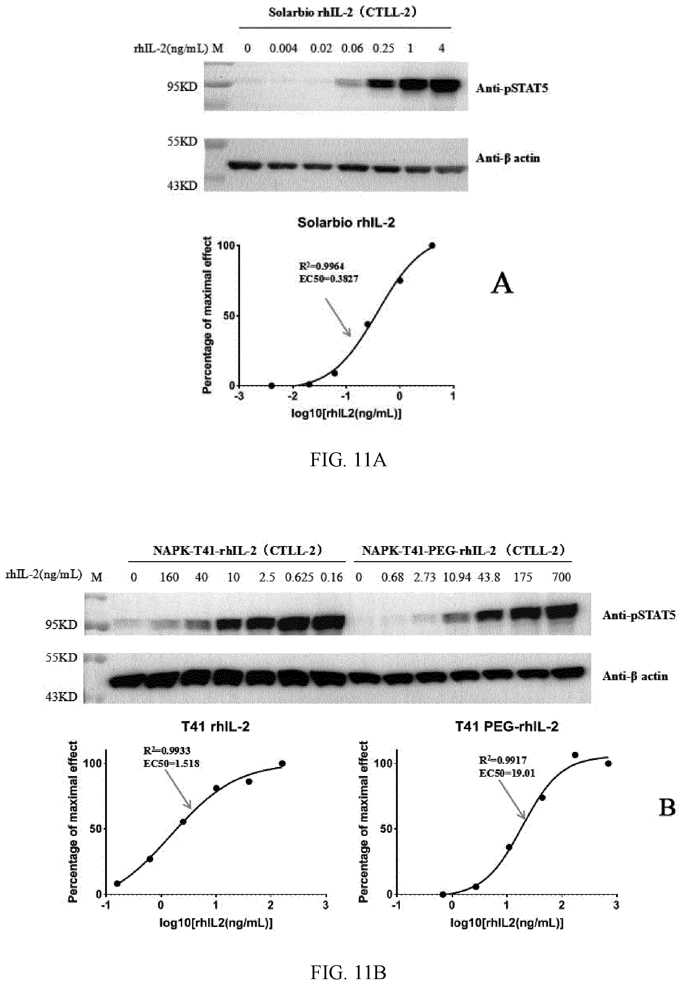

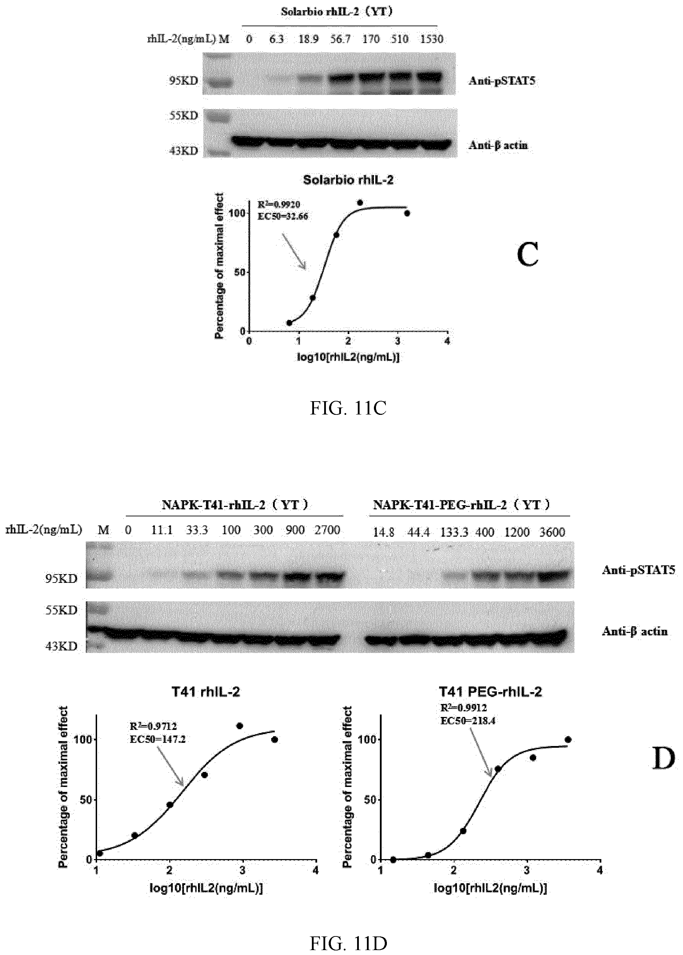

Results were shown in FIG. 11A to FIG. 11D and Table 6. The results showed that the 30KD PEG-rhIL2-T41NPAK met original design requirements (compared with the reference, the percentage change of EC50 ratio was lower than that of the reference).

**Table 6 Results of STAT5 phosphorylation assay**

| Name of sample | EC50 (ng/mL) of STATS phosphorylation assay (CTLL-2 cells) | | EC50 (ng/mL) of STATS phosphorylation assay (YT cells) | | Percentage change of EC50 ratio of YT/CTLL-2 cells of 30KD PEG-rhIL2 sample* |
|---|---|---|---|---|---|
| | rhIL-2 | 30KD PEG-rhIL2 | rhIL-2 | 30KD PEG-rhIL2 | |
| Reference | 0.38 | / | 32.7 | / | 100% |
| 30KD PEG-rhIL2-T41NPAK | 1.52 | 19.01 | 147.2 | 218.4 | 13.4% |

| | | | | | |
|---|---|---|---|---|---|
| *The calculation formula was the same as that of Example 5. | | | | | |

Unless otherwise specified, the terms used in the present invention have the meanings commonly understood by those skilled in the art.

The described embodiments of the present invention are for exemplary purposes only, and are not intended to limit the protection scope of the present invention. Those skilled in the art can make various other substitutions, changes and improvements within the scope of the present invention. Therefore, the present invention is not limited to the above embodiments, but only limited by the claims.

## Claims

1. A human interleukin 2-polyethylene glycol conjugate, comprising a recombinant human interleukin 2 containing at least one unnatural amino acid and PEG coupled to the at least one unnatural amino acid;
the unnatural amino acid is a compound containing a carbonyl terminal group and having a structure as shown in formula (I) or an enantiomer thereof, and the PEG is coupled to the at least one unnatural amino acid by forming an oxime bond between the carbonyl terminal group and the PEG containing a hydroxylamine terminal group, 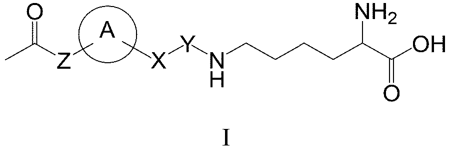
wherein X and Z each independently represent substituted or unsubstituted C0-C20 linear or branched alkylene, one or more -CH₂- are optionally substituted by one or more of -O-, -S-, -NH-, -C(O)- and -S(O)-, Y represents -C(O)-, -S(O)- or -CH₂-, and A represents substituted or unsubstituted C6-C20 aryl; and
when X, Z and A each independently represent a substituent, and the substituent is selected from one or more of hydroxyl, sulfhydryl, halogen, nitro, cyano, alkyl, alkenyl, alkynyl, alkoxy, acyl, acylamino, carboxyl, ester, amino, sulfonyl, sulfinyl, cycloalkyl, heterocyclyl, aryl and heteroaryl.

2. The human interleukin 2-polyethylene glycol conjugate according to claim 1, wherein the recombinant human interleukin 2 is a protein shown in SEQ ID NO: 3 or a functional active fragment thereof; and
preferably, in the recombinant human interleukin 2 containing at least one unnatural amino acid, a position of the at least one unnatural amino acid is selected from one or more sites corresponding to sites P34, K35, T37, R38, L40, T41, F42, K43, F44, Y45, E61, E62, K64, P65, E67, E68, N71, L72 and Y107 of SEQ ID NO: 2.

3. The human interleukin 2-polyethylene glycol conjugate according to claim 1 or 2, wherein the substituent is selected from one or more of hydroxyl, sulfhydryl, halogen, nitro, cyano, C1-C6 alkyl, C1-C6 alkoxy, acyl, acylamino, carboxyl, ester, amino, sulfonyl, sulfinyl, C3-C8 cycloalkyl, C3-C8 heterocyclyl, C6-C20 aryl and C4-C10 heteroaryl;
preferably, X and Z each independently represent C0-C10 linear or branched alkylene, and preferably represent C0-C6 linear alkylene, and one or more -CH₂- are optionally substituted by one or more of -O-, -S- and -NH-; and more preferably, X and Z are not C0 alkylene simultaneously; and/or
A represents substituted or unsubstituted C6-C10 aryl, and more preferably, A represents substituted or unsubstituted phenyl or naphthyl.

4. The human interleukin 2-polyethylene glycol conjugate according to any one of claims 1-3, wherein the unnatural amino acid is a compound with a structure as shown in formula (I-1), 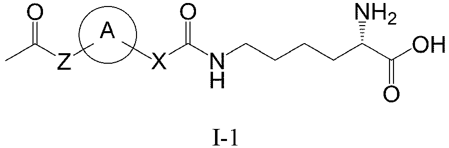
wherein X, Y and A are each independently as defined in any one of claims 1-3;
preferably, the unnatural amino acid is a compound with a structure as shown in formula (I-2), 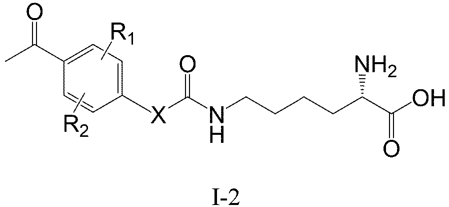
wherein X is as defined in any one of claims 1-3; and
R₁ and R₂ each independently represent hydrogen, hydroxyl, sulfhydryl, halogen, nitro, cyano, C1-C6 alkyl, C1-C6 alkoxy, acyl, acylamino, carboxyl, ester, amino, sulfonyl, sulfinyl, C3-C8 cycloalkyl, C3-C8 heterocyclyl, C6-C20 aryl or C4-C10 heteroaryl.

5. The human interleukin 2-polyethylene glycol conjugate according to claim 4, wherein the unnatural amino acid is a compound with a structure as shown in formula (I-3), formula (I-4), formula (I-5) or formula (I-6), 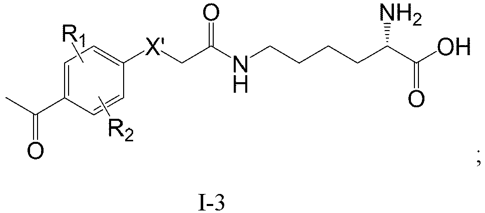 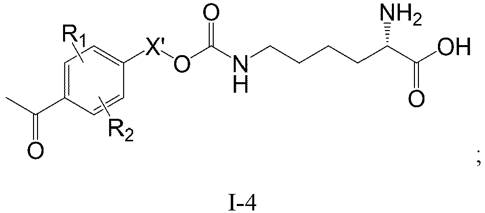 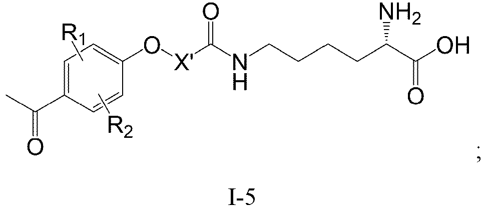 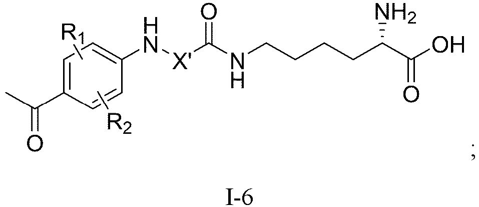
wherein X' represents C0-C6 linear alkylene, and preferably represents C0-C4 linear alkylene, and one or more -CH₂- are optionally substituted by -O- and/or -NH-; and
R₁ and R₂ are each independently as defined in claim 4.

6. The human interleukin 2-polyethylene glycol conjugate according to claim 5, wherein the unnatural amino acid is a compound with one of the structures as follows,
| | |
|---|---|
| (1) | 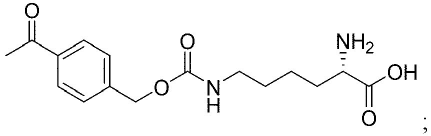 |
| (2) | 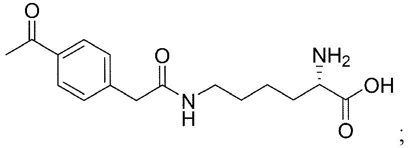 |
| (3) | 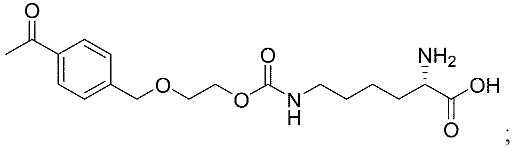 |
| (4) | 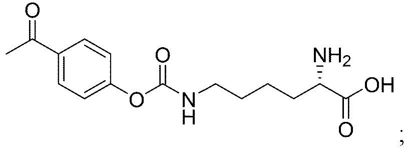 |
| (5) | 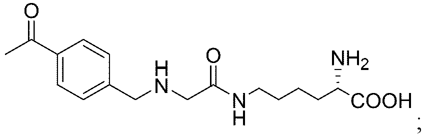 |
| (6) | 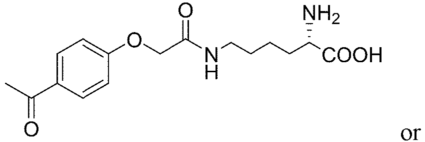 |
| (7) | 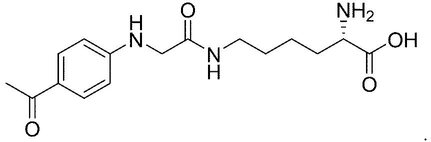 |

7. The human interleukin 2-polyethylene glycol conjugate according to any one of claims 1-6, wherein the PEG containing the hydroxylamine terminal group has a molecular weight of 20 KD to 50 KD.

8. The human interleukin 2-polyethylene glycol conjugate according to any one of claims 1-7, wherein the recombinant human interleukin 2 containing at least one unnatural amino acid is prepared by a codon expansion technology or by means of chemical synthesis.

9. The human interleukin 2-polyethylene glycol conjugate according to claim 8, wherein the codon expansion technology is implemented in *Escherichia coli.*

10. Use of the human interleukin 2-polyethylene glycol conjugate according to any one of claims 1-9 in preparation of a drug for promoting immunity, preventing and/or treating solid tumors and hematologic tumors, and/or expanding CD8⁺ T cells;
preferably, the solid tumors refer to bladder cancer, bone cancer, brain cancer, breast cancer, colorectal cancer, esophageal cancer, ocular cancer, head and neck cancer, kidney cancer, lung cancer, melanoma, ovarian cancer, pancreatic cancer or prostate cancer; and
preferably, the hematologic tumors refer to chronic lymphocytic leukemia (CLL), small lymphocytic lymphoma (SLL), follicular lymphoma (FL), diffuse large B-cell lymphoma (DLBCL), mantle cell lymphoma (MCL), Waldenstrom's macroglobulinemia, multiple myeloma, extranodal marginal zone B-cell lymphoma, nodal marginal zone B-cell lymphoma, Burkitt lymphoma, non-Burkitt high-grade B-cell lymphoma, primary mediastinal B-cell lymphoma (PMBL), immunoblastic large cell lymphoma, precursor B-cell lymphoblastic lymphoma, B-cell prolymphocytic leukemia, lymphplasmacytic lymphoma, splenic marginal zone lymphoma, plasma cell myeloma, plasmacytoma, mediastinal (thymic) large B-cell lymphoma, intravascular large B-cell lymphoma, primary effusion lymphoma, or lymphomatoid granulomatosis.
